# EUROPEAN PATENT SPECIFICATION

(11) **EP 2 609 114 B1**
(45) Date of publication and mention of the grant of the patent: **11.04.2018**
(21) Application number: 11749323.9
(22) Date of filing: 24.08.2011
(51) Int. Cl.: C07K 16/10, C12N 15/13, G01N 33/53

(54) **HIV CORE PROTEIN SPECIFIC ANTIBODIES AND USES THEREOF**
ANTIKÖRPER SPEZIFISCH FÜR DAS HIV CORE PROTEIN UND DEREN VERWENDUNG
ANTICORPS SPECIFIQUES DIRIGÉS CONTRE LE PROTÉINE DU NOYAU DU HIV AND LEUR UTILISATION

(30) Priority: 24.08.2010 US 376590 P
(43) Date of publication of application: 03.07.2013
(73) Proprietor: ABBOTT LABORATORIES, Abbott Park, IL 60064-3500 (US)
(72) Inventor: BELK, Jonathan, Grantham, New Hampshire 03753 (US); BROPHY, Susan, Lindenhurst Illinois 60046 (US); HUANG, Dagang, Shanghai, 201906 (CN); TIEMAN, Bryan, Elmhurst Illinois 60126 (US); SCHEFFEL, James, Shelby North Carolina 28150 (US); TYNER, Joan, Beach Park Illinois 60087 (US); ZIEMANN, Robert, Palatine Illinois 60067 (US)
(74) Representative: Modiano, Micaela Nadia
(86) International application number: PCT/US2011/048910
(87) International publication number: WO 2012/027440

(56) References cited:
- WO-A2-02/064615
- HAARD DE H J W ET AL: "SELECTION OF RECOMBINANT, LIBRARY-DERIVED ANTIBODY FRAGMENTS AGAINST P24 FOR HUMAN IMMUNODEFICIENCY VIRUS TYPE 1 DIAGNOSTICS", CLINICAL AND DIAGNOSTIC LABORATORY IMMUNOLOGY, AMERICAN SOCIETY FOR MICROBIOLOGY, US, vol. 5, no. 5, 1 September 1998 (1998-09-01), pages 636-644, XP000939091, ISSN: 1071-412X
- LEVIN A M ET AL: "Optimizing the affinity and specificity of proteins with molecular display", MOLECULAR BIOSYSTEMS, vol. 2, no. 1, January 2006 (2006-01), pages 49-57, XP002665432, ISSN: 1742-206X(print)

## Description

### CROSS-REFERENCE TO RELATED APPLICATIONS

This application claims priority to U.S. Patent Application No. 61/376,590 filed August 24, 2010.

### FIELD OF THE INVENTION

The present invention relates to antibodies that specifically bind to Human Immunodeficiency Virus-1 (HIV-1) and Human Immunodeficiency Virus (HIV-2) core antigen(s) with a high binding affinity, methods for producing and selecting said antibodies, immunoassays for the detection of Human Immunodeficiency Virus (HIV) that employ these antibodies and therapeutic compositions containing these antibodies.

### BACKGROUND

Human Immunodeficiency Virus (HIV) is a lentivirus (a member of the retrovirus family) (Gonda et al. (1985) Science, 227: 173-177; Stephan et al. (1986) Science, 231: 589-594). Infection with HIV can lead to Acquired Immunodeficiency Syndrome (AIDS). Transmission of HIV usually occurs through unprotected sexual intercourse, contaminated needles, breast milk, and an infected mother to her baby at birth.

HIV is a retrovirus having two copies of positive single-stranded RNA enclosed by a conical capsid. P24 is the major structural protein of the capsid with approximately 2000 copies of p24 per capsid. (McGovern et al. (2002) J. Med. Chem., 45(8): 1712-1722). The virus genome contains several genes including *gag, pol* (encoding polymerase), *env* (encoding envelope glycoprotein) genes. The *gag* gene encodes a core precursor named Pr55^{Gag}, which is cleaved into p17 (myristilated gag protein), p24 (major structural protein), p7 (nucleic acid binding protein), and p9 (proline-rich protein).

The most common marker for HIV antigen diagnosis is the viral structure proteins. As the major capsid structural protein, the p24 antigen test is widely used in HIV antigen test along with antibody test to help diagnose early HIV infection. Levels of p24 antigen increase significantly in 1 to 3 weeks after infection.

Major component proteins from HIV-1 Group M, HIV-1 Group O, and HIV-2 viruses are antigenically similar because of high amino acid sequence homology. The core proteins of HIV-1 Group M, HIV-1 Group O, and HIV-2 are related but not identical. Determination of HIV genetic variability has not been predicted but has relied on empirical observations. A shared epitope between HIV species and subtypes may or may not exist. HIV 115B-151-423 monoclonal antibody (mAb) specifically binds to HIV-1 Group M and HIV-1 Group O, and cross-react to HIV-2.

Ligand binding assays, such as immunoassays for measuring p24 antigen are commercially available. The current HIV Combo Assay on both ARCHITECT® and PRISM® instruments (Abbott Laboratories, Abbott Park, IL) utilizes HIV 115B-151-423 monoclonal antibody (mAb) as the conjugate in a sandwich format immunoassay for p24 antigen detection. HIV 115B-151-423 mAb specifically binds to HIV-1 Group M and HIV-1 Group O, and is cross-reactive to HIV-2.

U.S. Patent No. 7,531,642 (hereinafter referred to as the "'642" patent) discloses isolated HIV antibodies that are monospecifically reactive to various epitopes on p24. More particularly, the '642 patent describes HIV 115-151-423 isolated monoclonal antibodies. The HIV 115-151-423 monoclonal antibody is produced by the cell line having A.T.C.C. Deposit No. PTA-2809. The '642 patent also describes the use of the antibodies in immunoassays for the purpose of determining the presence of one or more antigens selected from the group consisting of HIV-1 antigen and HIV-2 antigen in a biological sample.

Although the current most predominant HIV immunoassay has a high sensitivity with a detection limit of approximately 10 pg of p24 antigen per 1 mL of patient sample (Abbott HIV Ab/Ag Combo Assay), its sensitivity is still 500∼1000-fold lower than nucleic acid testing (NAT) (Johanson et al. (2001) J. Virological Meth., 95: 81-92). Abbott HIV Ab/Ag Combo Assay incubates patient samples with microsphere for 18 minutes. Some other publications reported a higher sensitivity of ∼1 pg/ml (Ondoa et al. (2009) Cytometry Part B (Clinical Cytometry) 76B: 231-236; Ishikawa et al. (1998) J. Clin. Lab. Anal., 12(6): 343-350) with a much longer incubation time, such as 20 hours (Ishikawa et al. (1998) *supra*). Among all the means that are being studied to increase immunoassay sensitivity, increasing conjugate antibody's antigen binding affinity can directly lead to sensitivity improvement by binding more antigens within a specific timeframe. An immunoassay with improved sensitivity can rival the NAT with the advantages including faster sample processing time and lower cost.

The specificity and sensitivity of the antibodies used in immunoassays, such as HIV immunoassays, are very important. One way in which to increase both the specificity and sensitivity of one or more antibodies is to improve the binding affinity of an antibody for its intended target (*i.e.,* an antigen). Antibodies having an improved binding affinity for their intended targets should exhibit increased specificity and sensitivity.

### SUMMARY

This invention pertains to the identification of new antibodies that bind HIV core antigens. In certain aspects, an isolated antibody is provided that binds to an HIV-I and/or HIV-2 core protein the antibody exhibiting a greater antigen binding affinity than the HIV 115B-151-423 mAb to HIV-1 core protein (p24) and/or to HIV-2 core protein (p26). In certain embodiments the antibody immunospecifically binds to p24 with an equilibrium dissociation constant (K_{D}) of less than 7 pM, less than about 5 pM, more preferably less than about 1 pM. In various aspects the antibody comprises a light chain variable domain comprising complementarity determining regions L1, L2 and L3, and a heavy chain variable domain comprising complementarity determining regions H1, H2 and H3 where the amino acid sequences of one or more of the L1, L2, or L3 CDRs of the light chain variable domain comprise the amino acid sequences respectively of the L1, L2, and/or L3 CDRs of an antibody selected from the group consisting of 115B-151-423 AM1 and 115B-151-423 AM2; and/or the amino acid sequences of one or more of the H1, H2, or H3 CDRs of the heavy chain variable domain comprise the amino acid sequences respectively of the H1, H2, and/or H3 CDRs of an antibody selected from the group consisting of 115B-151-423 AM1 and 115B-151-423 AM2. In certain aspects the L3 CDR of the antibody comprises the amino acid sequence of the L3 CDR of an antibody selected from the group consisting of 115B-151-423 AM1 and 115B-151-423 AM2; and/or the H2 and/or H3 CDRs of the antibody comprise the amino acid sequences respectively of the H2 and H3 CDRs of an antibody selected from the group consisting of 115B-151-423 AM1 and 115B-151-423 AM2. In certain aspects the H2 and H3 CDRs of the antibody comprise the amino acid sequences respectively of the H2 and H3 CDRs of an antibody selected from the group consisting of 115B-151-423 AM1 and 115B-151-423 AM2. In certain aspects the H1, H2 and H3 CDRs of the antibody comprise the amino acid sequences respectively of the H1, H2 and H3 CDRs of an antibody selected from the group consisting of 115B-151-423 AM1 and 115B-151-423 AM2; and/or the L1, L2 and L3 CDRs of the antibody comprise the amino acid sequences respectively of the L1, L2 and L3 CDRs of an antibody selected from the group consisting of 115B-151-423 AM1 and 115B-151-423 AM2. In certain aspects the H1, H2 and H3 CDRs of the antibody comprise the amino acid sequences respectively of the H1, H2 and H3 CDRs of an antibody selected from the group consisting of 115B-151-423 AM1 and 115B-151-423 AM2. In certain aspects the L1, L2 and L3 CDRs of the antibody comprise the amino acid sequences respectively of the L1, L2 and L3 CDRs of an antibody selected from the group consisting of 115B-151-423 AM1 and 115B-151-423 AM2. In certain aspects the H1, H2 and H3 CDRs of the antibody comprise the amino acid sequences respectively of the H1, H2 and H3 CDRs of 115B-151-423 AM1; and the L1, L2 and L3 CDRs of the antibody comprise the amino acid sequences respectively of the L1, L2 and L3 CDRs of 115B-151-423 AM1. In certain aspects the H1, H2 and H3 CDRs of the antibody comprise the amino acid sequences respectively of the HI, H2 and H3 CDRs of 115B-151-423 AM2; and the L1, L2 and L3 CDRs of the antibody comprise the amino acid sequences respectively of the L1, L2 and L3 CDRs of 115B-151-423 AM2.

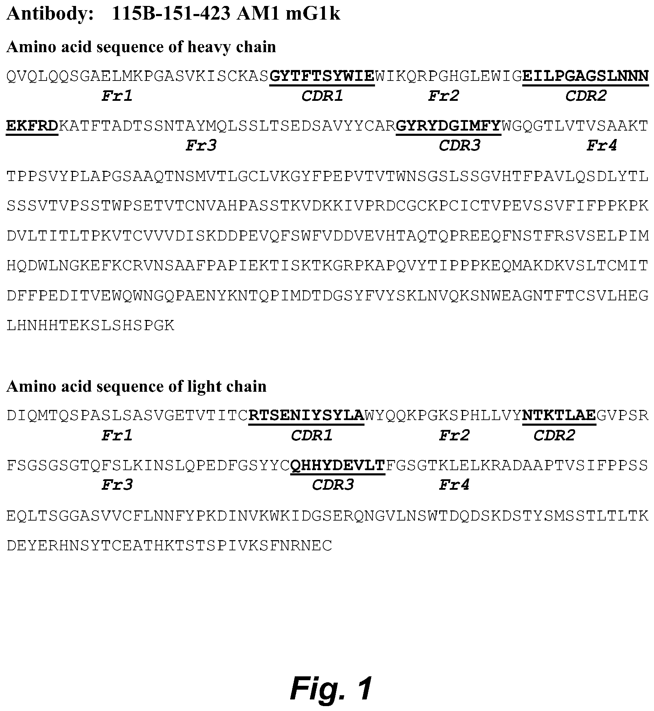

In various embodiments the antibody is selected from the group consisting of monoclonal antibody, a multispecific antibody, an animal antibody, and a recombinant In certain embodiments the amino acid sequence of the heavy chain of the antibody comprises a heavy chain sequence shown in Figures 1-4. In certain embodiments the amino acid sequence of the light chain of the antibody comprises a light chain sequence shown in Figures 1-4. In certain embodiments the amino acid sequence of the heavy chain of the antibody comprises a heavy chain sequence shown in Figures 1-4; and the amino acid sequence of the light chain of the antibody comprises a light chain sequence shown in Figures 1-4 In certain embodiments the antibody is a mouse monoclonal antibody. In certain embodiments the antibody is a mouse-human chimeric antibody. In certain embodiments the antibody is attached to a solid substrate.

In various aspects nucleic acids are provided where the nucleic acids encode any one or more of the antibodies described herein. In various aspects the nucleic acid is in an expression cassette. Cells transfected with such nucleic acids are disclosed where the cells express the antibody encoded by the nucleic acid. In certain embodiments the cell is a cell selected from the group consisting of a mammalian cell, an insect cell, and a fungal cell. In certain aspects the cell is a mammalian cell (*e.g.,* a CHO cell).

In various embodiments methods are provided for detecting the presence of one or more antigens selected from the group consisting of HIV-1 antigen and HIV-2 antigen, in a test sample. The methods typically involve contacting the test sample with at least one antibody described herein that binds to Human Immunodeficiency Virus-1 protein p24 and/or Human Immunodeficiency Virus-2 protein p26 for a time and under conditions sufficient for the formation of an antibody/antigen complex; and detecting the antibody/antigen complex, where the presence of the complex indicates the presence of at least HIV-1 or HIV2 antigen in the test sample. In certain embodiments the antibody is labeled and the detecting comprises detecting the label. In certain embodiments the method further involves contacting a conjugate to the antibody/antigen complex for a time and under conditions sufficient to allow the conjugate to bind to the bound antigen comprising the complex, where the conjugate comprises a second antibody attached to a signal generating compound capable of generating a detectable signal; and detecting presence of antigen present in the test sample by detecting a signal generated by the signal generating compound, the presence or strength of the signal indicating the presence of at least one antigen selected from the group consisting of HIV-1 antigen and HIV-2 antigen in the test sample.

In certain embodiments methods are provided for detecting the presence of one or more antigens selected from the group consisting of HIV-1 antigen and HIV-2 antigen, in a test sample where the methods involve contacting the test sample with at least one first antibody that binds to Human Immunodeficiency Virus-1 protein p24 and/or Human Immunodeficiency Virus-2 protein p26 for a time and under conditions sufficient for the formation of an antibody/antigen complex; and adding a conjugate to the resulting antibody/antigen complex for a time and under conditions sufficient to allow the conjugate to bind to the bound antigen, where the conjugate comprises an antibody as described herein attached (or attachable to) to a signal generating compound capable of generating a detectable signal; and detecting presence of antigen which may be present in the test sample by detecting a signal generated by the signal generating compound, the presence or quantity of the signal indicating the presence of at least one antigen selected from the group consisting of HIV-1 antigen and HIV-2 antigen in the test sample.

In certain aspects methods are provided for detecting the presence of one or more antigens selected from the group consisting of HIV-1 antigen and HIV-2 antigen, in a test sample where the methods involve contacting: 1) a first antibody that binds to HIV-1 p24 antigen and/or HIV-2 p26 antigen bound to a solid support, 2) the test sample, and 3) an indicator reagent comprising a second antibody that binds to HIV-1 p24 antigen and/or HIV-2 p26 antigen to which a signal generating compound is attached, to form a mixture, where the first antibody and/or the second antibody is an antibody described herein; incubating the mixture for a time and under conditions sufficient to form an antibody/antigen/antibody complex; and detecting presence of a measurable signal generating by the signal-generating compound, the presence or quantity of the signal indicating presence of one or more antigens in the test sample selected from the group consisting of HIV-1 antigen and HIV-2 antigen. In certain aspects the first antibody and/or the second antibody is an antibody described herein.

In various embodiments kits are provided for detecting the presence of one or more antigens selected from the group consisting of HIV-1 antigen and HIV-2 antigen, in a test sample. In certain embodiments the kits comprise a first antibody that binds to HIV-1 p24 antigen and/or HIV-2 p26 antigen; and/or an indicator reagent comprising a second antibody that binds to HIV-1 p24 antigen and/or HIV-2 p26 antigen to which a signal generating compound is attached; where the first antibody and/or the second antibody is an antibody described herein.

### DEFINITIONS

The terms "polypeptide", "peptide" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The term also includes variants on the traditional peptide linkage joining the amino acids making up the polypeptide. Preferred "peptides", "polypeptides", and "proteins" are chains of amino acids whose alpha carbons are linked through peptide bonds. In certain embodiments the amino acid residues comprising the peptide are "L-form" amino acid residues, however, it is recognized that in various embodiments, "D" amino acids can be incorporated into the peptide. Peptides also include amino acid polymers in which one or more amino acid residues is an artificial chemical analogue of a corresponding naturally occurring amino acid, as well as to naturally occurring amino acid polymers. In addition, the term applies to amino acids joined by a peptide linkage or by other, "modified linkages" (*e.g*., where the peptide bond is replaced by an α-ester, a β-ester, a thioamide, phosphonamide, carbomate, hydroxylate, and the like (*see, e.g.,* Spatola et al. (1983) Chem. Biochem. Amino Acids and Proteins 7: 267-357), where the amide is replaced with a saturated amine (*see, e.g.,* Skiles et al., U.S. Pat. No. 4,496,542, which is incorporated herein by reference, and Kaltenbronn et al., (1990) Pp. 969-970 in Proc. 11th American Peptide Symposium, ESCOM Science Publishers, The Netherlands, and the like)).

As used herein, an "antibody" refers to a protein consisting of one or more polypeptides substantially encoded by immunoglobulin genes or fragments of immunoglobulin genes or proteins derived from such "precursors" (*e.g*., as by affinity maturation, chain shuffling, *etc.*). The recognized immunoglobulin genes include the kappa, lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively.

As used herein, the terms "antibody" and "antibodies" include polyclonal antibodies, monoclonal antibodies, multispecific antibodies, human antibodies, humanized antibodies (fully or partially humanized), animal antibodies (*e.g*., derived from a mammal, including a non-primate (for example, a cow, pig, camel, llama, horse, goat, rabbit, sheep, hamsters, guinea pig, cat, dog, rat, mouse), a non-human primate (for example, a monkey, such as a cynomolgus monkey, a chimpanzee, *etc.*), recombinant antibodies, chimeric antibodies, single-chain Fvs (scFv), single chain antibodies, single domain antibodies, Fab fragments, F(ab')₂ fragments, disulfide-linked Fv (sdFv), chemically conjugated Fv (ccFv), and anti-idiotypic (anti-Id) antibodies (including, for example, anti-Id antibodies to antibodies of the present invention), and functionally active epitope-binding fragments of any of the above. In certain embodiments antibodies also include affibodies, nanobodies, and unibodies. In certain embodiments particular, antibodies include immunoglobulin molecules and immunologically active fragments of immunoglobulin molecules, namely, molecules that contain an antigen binding site. Immunoglobulin molecules can be of any type (for example, IgG, IgE, IgM, IgD, IgA and IgY), class (for example, IgG₁, IgG₂, IgG₃, IgG₄, IgA₁ and IgA₂) or subclass.

As used herein, the term "association rate", "kₒₙ" or "kₐ" as used interchangeably herein, refers to the value indicating the binding strength (degree) of an antibody to its target antigen or the rate of complex formation between mAb and antigen as shown by the below:

Antibody (Ab) + Antigen (Ag) → Ab-Ag

Methods for determining association constants (kₐ) are well known in the art. For example, surface plasmon resonance can be used, such as a BIACORE® (GE Healthcare, NJ) assay. Further, a KINEXA® (Kinetic Exclusion Assay) assay, available from Sapidyne Instruments (Boise, Idaho) can also be used.

As used herein, the term "dissociation rate", "k_{off}" or "k_{d}" as used interchangeably herein, refers to the value indicating the dissociation strength (degree) of an antibody from its target antigen or separation of Ab-Ag complex over time into free mAb and antigen as shown by the below:

Antibody (Ab) + Antigen (Ag) ← Ab-Ag

Methods for determining dissociation constants (k_{d}) are well known in the art. For example, surface plasmon resonance can be used, such as a BIACORE® (GE Healthcare, NJ) assay. Additionally, a KINEXA® (Kinetic Exclusion Assay) assay, available from Sapidyne Instruments (Boise, Idaho) can also be used.

As used herein, the term "equilibrium dissociation constant" or "K_{D}" as used interchangeably, herein, refers to the value obtained by dividing the dissociation rate (k_{off}) by the association rate (kₒₙ). The association rate, the dissociation rate and the equilibrium dissociation constant are used to represent the binding affinity of an antibody to an antigen.

As used herein, the term "epitope" or "epitopes" refers to sites or fragments of a polypeptide or protein having antigenic or immunogenic activity in a subject. An epitope having immunogenic activity is a site or fragment of a polypeptide or protein that elicits an antibody response in an animal. An epitope having antigenic activity is a site or fragment of a polypeptide or protein to which an antibody immunospecifically binds as determined by any method well-known to those skilled in the art, for example by immunoassays.

As used herein, the term "humanized" antibody refers to an immunoglobulin variant or fragment thereof, which is capable of binding to a predetermined antigen and which comprises framework regions having substantially the amino acid sequence of a human immunoglobulin and CDRs having substantially the amino acid sequence of a non-human immunoglobulin. Ordinarily, a humanized antibody has one or more amino acid residues introduced into it from a source that is non-human. In general, the humanized antibody will include substantially all of at least one, and typically two, variable domains (*e.g.,* Fab, Fab', F(ab')₂, Fv) in which all or substantially all of the CDR regions correspond to those of a non-human immunoglobulin and all or substantially all of the FR regions are those of a human immunoglobulin consensus sequence. The humanized antibody optimally comprises at least a portion of an immunoglobulin constant region (Fc), typically that of a human immunoglobulin. Generally, the antibody will contain both the light chain as well as at least the variable domain of a heavy chain. The humanized antibody can be selected from any class of immunoglobulins, including IgM, IgG, IgD, IgA and IgE, and any isotype, including IgG₁, IgG₂, IgG₃ and IgG₄. The humanized antibody may comprise sequences from more than one class or isotype, and selecting particular constant domains to optimize desired effector functions is within those skilled in the art.

As used herein, the phrase "immunospecifically binds to a p24 epitope", "immunospecifically binds to p24" and analogous terms thereof refer to peptides, polypeptides, proteins, fusion proteins and antibodies that specifically bind to p24 or p24 fragment and do not specifically bind to other peptides. The phrase "immunospecifically binds to a p26 epitope", "immunospecifically binds to p26" and analogous terms thereof refer to peptides, polypeptides, proteins, fusion proteins and antibodies that specifically bind to p26 or p26 fragment and do not specifically bind to other peptides. As used herein, the phrase "immunospecifically binds to a p24 epitope and/or to a p26 epitope", "immunospecifically binds to p24 and/or to p26" and analogous terms thereof refer to peptides, polypeptides, proteins, fusion proteins and antibodies that specifically bind to p24 or a p24 fragment or a p24 epitope and/or to p26, a p26 fragment and/or to a p26 epitope and do not specifically bind to other peptides. In certain embodiments a peptide, polypeptide, protein, or antibody that immunospecifically binds to a p24, p26, or p24 and/or p26 or fragments or epitopes thereof may bind to other peptides, polypeptides, or proteins with lower binding affinity as determined by, for example, immunoassays, BIAcore, or other assays known in the art.

As used herein, the term "isolated" in the context of nucleic acid molecules refers to a nucleic acid molecule which is separated from other nucleic acid molecules which are present in the natural source of the nucleic acid molecule. Moreover, an "isolated" nucleic acid molecule, such as a cDNA molecule, can be substantially free of other cellular material, or culture medium when produced by recombinant techniques, or substantially free of chemical precursors or other chemicals when chemically synthesized. In one aspect, nucleic acid molecules are isolated. In another aspect, a nucleic acid molecule encoding an antibody of the invention is isolated.

As used herein, the term "stringent conditions" refers to hybridization to filter-bound DNA in 6 x sodium chloride/sodium citrate (SSC) at about 45°C followed by one or more washes in 0.2 x SSC/0.1% SDS at about 50-65°C. The term "under highly stringent conditions", refers to hybridization to filter-bound nucleic acid in 6 x SSC at about 45°C followed by one or more washes in 0.1 x SSC/0.2% SDS at about 68°C, or under other stringent hybridization conditions which are known to those skilled in the art (see, for example, Ausubel et al., eds. (1989) Current Protocols in Molecular Biology, Vol. I, Green Publishing Associates, Inc. and John Wiley & Sons, Inc., New York at pages 6.3.1-6.3.6 and 2.10.3).

As used herein, the terms "subject" and "patient" are used interchangeably. As used herein, the terms "subject" and "subjects" refer to a non-human mammal (*e.g.,* canine, feline, porcine, ungulate, canine, lagomorph, non-human primate (for example, a monkey, such as a cynomolus monkey, chimpanzee)) or a human.

As used herein, the term "test sample" refers to a biological sample derived from a cell or tissue of an organism, preferably a mammal. Illustrative samples include, but are not limited to samples containing or derived from serum, plasma, whole blood, lymph, CNS fluid, urine, or other bodily fluids of a subject. The test sample can be prepared using routine techniques known to those skilled in the art.

As used herein, the term "therapeutically effective amount" or "pharmaceutically effective amount" means an amount of antibody or antibody portion effective, at dosages and for periods of time necessary, to achieve the desired therapeutic result. The exact dose will be ascertainable by one skilled in the art. As known in the art, adjustments based on age, body weight, sex, race, diet, time of administration, drug interaction and severity of condition may be necessary and will be ascertainable with routine experimentation by those skilled in the art. A therapeutically effective amount is also one in which the therapeutically beneficial effects outweigh any toxic or detrimental effects of the antibody or antibody fragment. A "prophylactically effective amount" refers to an amount effective, at dosages and for periods of time necessary to achieve the desired prophylactic result. Typically, since a prophylactic dose is used in subjects prior to or at an earlier stage of disease, the prophylactically effective amount will be less than the therapeutically effective amount.

### BRIEF DESCRIPTION OF THE DRAWINGS

Figure 1 shows the amino acid sequence of the heavy chain (VH) (SEQ ID NO:1) and light chain (VL) of antibody 115B-151-423 AM1 mG1k.
Figure 2 shows the amino acid sequence of the heavy chain (VH) (SEQ ID NO:2) and light chain (VL) of antibody 115B-151-423 AM2 mG1k.
Figure 3 shows the amino acid sequence of the heavy chain (VH) (SEQ ID NO:3) and light chain (VL) of antibody 115B-151-423 AM1 hG1k.
Figure 4 shows the amino acid sequence of the heavy chain (VH) (SEQ ID NO:4) and light chain (VL) of antibody 115B-151-423 AM2 hG1k.

### DETAILED DESCRIPTION

In certain aspects novel HIV antibodies are provided that show substantially higher binding affinity for HIV-1 and/or HIV-2 core proteins (p24 and/or p26). The antibodies were obtained by engineering mutations in the 115B-151-423 mAb (antibody secreted by cell line PTA-2809, see U.S. Patent 7,531,640) to produce antibodies showing approximately a 10-fold or greater improvement in antigen binding affinity.

Without being bound by a particular theory, it is believed that the new antibodies bind the same epitope(s) bound by the HIV 115B-151-423. More particularly, it is believed the new antibodies bind specifically binds to HIV-1 Group M and HIV-1 Group O, and cross-react to HIV-2 (*see, e.g.,* Example 1, herein).

The antibodies described herein have high affinities (K_{eq} values) sufficient to detect diagnostically relevant femtomolar quantities of HIV core protein. It is believed, however, that they also possess broad specificity (*i.e.,* shared-reactivity) for detection of equivalent quantities of related, but nonidentical, core proteins from HIV-1 Group M, HIV-1 Group O, and HIV-2.

The antibodies are well suited for the detection of HIV-1 Group M, HIV-1 Group O, and HIV-2 antigen(s) in a sample. The antibodies can also be used for the isolation or purification of HIV core proteins. The antibodies also find use in therapeutic and/or prophylactic applications.

### I. Antibodies.

As indicated above, in certain aspects novel isolated antibodies are provided that specifically bind (immunospecifically bind) to an HIV core protein. It is believed the antibodies bind to HIV-1 core protein (p24) and/or to HIV-2 core protein (p26). The antibodies show a substantially higher binding specificity to one or more target antigen(s) than the 115B-151-423 mAb described in U.S. Patent No:7,531,640 (produced by cell line PTA-2809). In certain aspects the antibodies bind HIV-1 core protein and/or HIV-2 core protein with an affinity at least about 1.5 fold, preferably at least about 2 fold, more preferably at least about 4 fold or at least about 8 fold, and most preferably with at least about 10 fold greater binding affinity than the HIV 115B-151-423 mAb. In certain embodiments the antibodies bind p24 and/or p26 with an with an equilibrium dissociation constant (K_{D}) of less than about 7 pM, , and in certain embodiments, with a K_{D} between about 7 pM and about 100 fM.

Also, in certain aspects compositions comprising the anti-HIV antibodies described herein attached to a substrate or to an effector (*e.g.,* a detectable label, a second antibody, a cytokine, *etc.*) are also provided. Where the effector comprise a second (or more) antibodies, a bispecific (or polyspecific) antibody is provided.

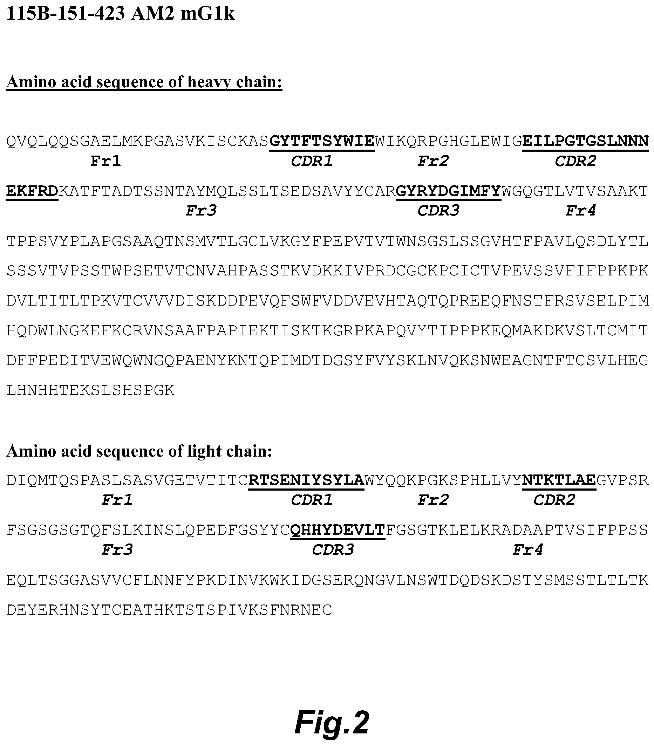

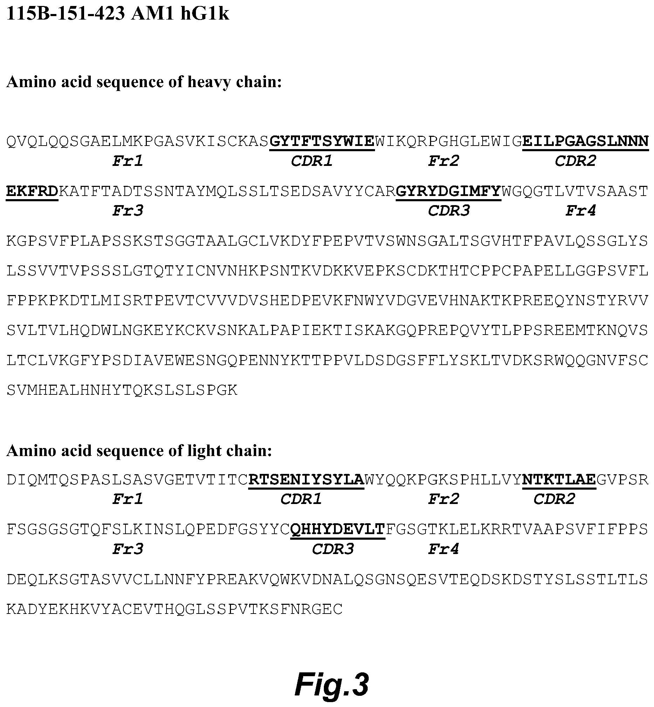

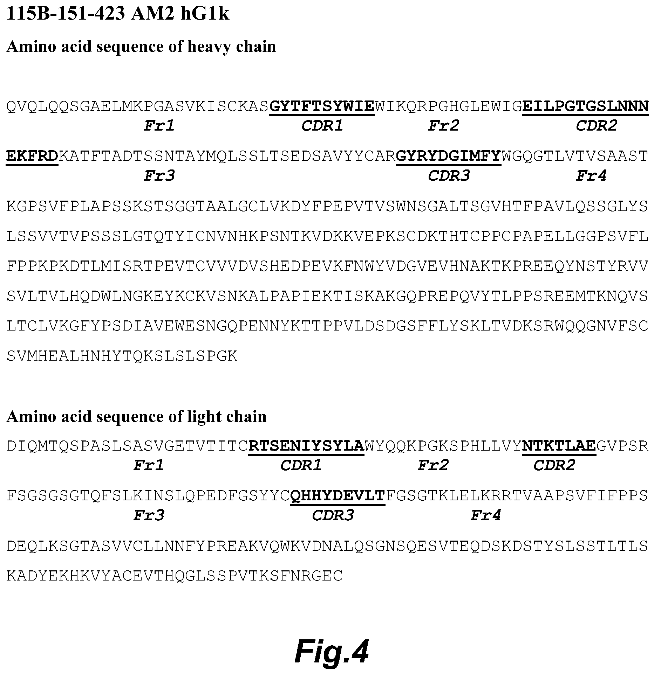

The heavy and light chain complementarity determining regions (CDRs) of two prototypic antibodies (115B-151-423 AM1 and 115B-151-423 AM2 CDR2) having substantially higher binding affinity for p24 and/or p26 than mAb 115B-151-423 are shown in Tables 1 and 2, respectively. The amino acid sequences of the entire VH and VL domains of these antibodies are shown in Figures 1-4. Notably, Figures 1 and 2, respectively, show the amino acid sequence of a murine and a mouse-human (IgG1 kappa) chimeric] AM1 antibody, while Figures 3 and 4, respectively show the amino acid sequence of a murine and a mouse-human (IgG1 kappa) chimeric] AM2 antibody

**Table 1. Amino acid sequence of heavy and light chain CDRs of 115B-151-423 AM1 antibody with comparison to wild type (115B-151-423 antibody (see, e.g., U.S. Patent 7,531,642).**

| **115B-151-423 AM1 CDRs** | **SEQ ID NO** |
|---|---|
| **CDR H1:** | |
| Gly-Tyr-Thr-Phe-Thr-Ser-Tyr-Trp-Ile-Glu (GYTFTSYWIE) | 5 |
| **CDR H2:** | |
| Glu-Ile-Leu-Pro-Gly-Ala-Gly-Ser-Leu-Asn-Asn-Asn- Glu-Lys-Phe-Arg-Asp (EILPGAGSLNNNEKFRD) | 6 |
| ***Wild-type CDR H2:*** | |
| Glu-Ile-Leu-Pro-Gly-Thr-Gly-Ser-Leu-Asn-Asn-Asn- Glu-Lys-Phe-Arg-Asp (EILPGTGSLNNNEKFRD) | 7 |
| **CDR H3:** | |
| Gly-Tyr-Arg-Tyr-Asp-Gly-Ile-Met-Phe-Tyr (GYRYDGIMFY) | 8 |
| ***Wild-type CDR H3:*** | |
| Gly-Tyr-Arg-Tyr-Asp-Gly-Trp-Phe-Ala-Tyr (GYRYDGWFAY) | 9 |
| **CDR L1:** | |
| Arg-Thr-Ser-Glu-Asn-Ile-Tyr-Ser-Tyr-Leu-Ala (RTSENIYSYLA) | 10 |
| **CDR L2:** | |
| Asn-Thr-Lys-Thr-Leu-Ala-Glu (NTKTLAE) | 11 |
| **CDR L3:** | |
| Gln-His-His-Tyr-Asp-**Glu-Val**-Leu-Thr (QHHYD**EV**LT) | 12 |
| **Wild-type *CDR L3:*** | |
| Gln-His-His-Tyr-Asp-**Ser-Pro**-Leu-Thr (QHHYD**SP**LT) | 13 |

**Table 2. Amino acid sequences of heavy and light chain CDRs of 115B-151-423 AM2 antibody with comparison to wildtype (115B-151-423 mAb)**

| **115B-151-423 AM2 CDR2** | **Seq ID No** |
|---|---|
| **CDR H1**: | |
| Gly-Tyr-Thr-Phe-Thr-Ser-Tyr-Trp-Ile-Glu (GYTFTSYWIE) | 14 |
| **CDR H2:** | |
| Glu-Ile-Leu-Pro-Gly-Thr-Gly-Ser-Leu-Asn-Asn-Asn- Glu-Lys-Phe-Arg-Asp (EILPGTGSLNNNEKFRD) | 15 |
| **CDR H3:** | |
| Gly-Tyr-Arg-Tyr-Asp-Gly-**Ile-Met-Phe**-Tyr (GYRYDG**IMF**Y) | 16 |
| *Wild-type CDR H3:* | |
| Gly-Tyr-Arg-Tyr-Asp-Gly-Trp-Phe-Ala-Tyr (GYRYDGWFAY) | 17 |
| **CDR L1:** | |
| Arg-Thr-Ser-Glu-Asn-Ile-Tyr-Ser-Tyr-Leu-Ala (RTSENIYSYLA) | 18 |
| **CDR L2:** | |
| Asn-Thr-Lys-Thr-Leu-Ala-Glu (NTKTLAE) | 19 |
| **CDR L3:** | |
| Gln-His-His-Tyr-Asp-**Glu-Val**-Leu-Thr (QHHYD**EV**LT) | 20 |
| ***Wild-type CDR L3:*** | |
| Gln-His-His-Tyr-Asp-**Ser-Pro**-Leu-Thr (QHHYD**SP**LT) | 21 |

Accordingly, in certain aspects antibodies are contemplated where the antibody comprises a light chain variable domain comprising complementarity determining regions L1, L2 and L3, and a heavy chain variable domain comprising complementarity determining regions HI, H2 and H3 wherein: the amino acid sequences of one or more of the L1, L2, or L3 CDRs of the light chain variable domain comprise the amino acid sequences respectively of the L1, L2, and/or L3 CDRs of an antibody selected from the group consisting of 115B-151-423 AM1 and 115B-151-423 AM2; and/or the amino acid sequences of one or more of the H1, H2, or H3 CDRs of the heavy chain variable domain comprise the amino acid sequences respectively of the H1, H2, and/or H3 CDRs of an antibody selected from the group consisting of 115B-151-423 AM1 and 115B-151-423 AM2. In certain aspects the L3 CDR of the antibody comprises the amino acid sequence of the L3 CDR of an antibody selected from the group consisting of 115B-151-423 AM1 and 115B-151-423 AM2; and/or the H2 and/or H3 CDRs of the antibody comprise the amino acid sequences respectively of the H2 and H3 CDRs of an antibody selected from the group consisting of 115B-151-423 AM1 and 115B-151-423 AM2.

In certain aspects the H2 and H3 CDRs of the antibody comprise the amino acid sequences respectively of the H2 and H3 CDRs of an antibody selected from the group consisting of 115B-151-423 AM1 and 115B-151-423 AM2. In certain aspects the H1, H2 and H3 CDRs of the antibody comprise the amino acid sequences respectively of the HI, H2 and H3 CDRs of an antibody selected from the group consisting of 115B-151-423 AM1 and 115B-151-423 AM2; and/or the L1, L2 and L3 CDRs of the antibody comprise the amino acid sequences respectively of the L1, L2 and L3 CDRs of an antibody selected from the group consisting of 115B-151-423 AM1 and 115B-151-423 AM2.

In certain aspects H1, H2 and H3 CDRs of the antibody comprise the amino acid sequences respectively of the H1, H2 and H3 CDRs of an antibody selected from the group consisting of 115B-151-423 AM1 and 115B-151-423 AM2.

In certain aspects the L1, L2 and L3 CDRs of the antibody comprise the amino acid sequences respectively of the L1, L2 and L3 CDRs of an antibody selected from the group consisting of 115B-151-423 AM1 and 115B-151-423 AM2.

In certain aspects the H1, H2 and H3 CDRs of the antibody comprise the amino acid sequences respectively of the H1, H2 and H3 CDRs of 115B-151-423 AM1; and the L1, L2 and L3 CDRs of the antibody comprise the amino acid sequences respectively of the L1, L2 and L3 CDRs of 115B-151-423 AM1.

In certain aspects the H1, H2 and H3 CDRs of the antibody comprise the amino acid sequences respectively of the H1, H2 and H3 CDRs of 115B-151-423 AM2; and the L1, L2 and L3 CDRs of the antibody comprise the amino acid sequences respectively of the L1, L2 and L3 CDRs of 115B-151-423 AM2.

In various aspects the antibody is selected from the group consisting of monoclonal antibody, a multispecific antibody, a human antibody, a fully humanized antibody, a partially humanized antibody, an animal antibody, a recombinant antibody, a chimeric antibody, a single chain antibody, a single-chain Fv, a single domain antibody, a Fab fragment, a F(ab')₂ fragment, a disulfide-linked Fv, an anti-idiotypic antibody, and a functionally active epitope-binding fragment thereof.

In various aspects the antibody is an Fv antibody where the variable heavy (VH) and variable light (VL) domains are either chemically conjugated (*e.g*., via a PEG or other linker) or are a single chain protein (scFv) and the VH and VL domains are joined by a peptide linker. In certain aspects the linker ranges in length from about 1. 2, or 3 amino acids to about 30, 40, or 50 amino acids. In certain aspects the peptide linker ranges in length from about 4 to about 30, preferably from about 8 to about 24 amino acids in length. Illustrative linkers include, but are not limited to the peptide linkers shown in Table 3.

**Table 3. Illustrative peptide linkers for joining VH and VL domains in a single-chain peptide.**

| **Linker** | **Seq ID NO** |
|---|---|
| GGGGS | 22 |
| GGGGS GGGGS | 23 |
| GGGGS GGGGS GGGGS | 24 |
| GGGGS GGGGS GGGGS GGGGS | 25 |
| GPAKELTPLKEAKVS | 26 |
| GSTSGSGKSSEGKG | 27 |
| RPLSYRPPFPFGFPSVRP | 28 |
| YPRSIYIRRRHPSPSLTT | 29 |
| TPSHLSHILPSFGLPTFN | 30 |
| RPVSPFTFPRLSNSWLPA | 31 |
| SPAAHFPRSIPRPGPIRT | 32 |
| APGPSAPSHRSLPSRAFG | 33 |
| PRNSIHFLHPLLVAPLGA | 34 |
| MPSLSGVLQVRYLSPPDL | 35 |
| SPQYPSPLTLTLPPHPSL | 36 |
| NPSLNPPSYLHRAPSRIS | 37 |
| LPWRTSLLPSLPLRRRPS | 38 |
| PPLFAKGPVGLLSRSFPP | 39 |
| VPPAPVVSLRSAHARPPY | 40 |
| LRPTPPRVRSYTCCPTP | 41 |
| PNVAHVLPLLTVPWDNLR | 42 |
| CNPLLPLCARSPAVRTFP | 43 |

In certain aspects derived or variant antibodies are contemplated that comprises at least one mutation (such as deletions, additions and/or substitutions) in at least one of the heavy chain complementary determining ("CDR") regions (for example, the heavy chain CDR 1, heavy chain CDR 2, or heavy chain CDR 3), at least one mutation (such as deletions, additions and/or substitutions) in the light chain CDR regions (for example, the light chain CDR 1, light chain CDR 2, or light chain CDR 3) when compared to the amino acid sequence of the antibodies whose CDRs are shown in Tables 1 and 2 and/or whose heavy and light chains are shown in Figures 1-4. Standard techniques known to those of skill in the art can be used to introduce mutations (such as deletions, additions, and/or substitutions) in the nucleic acid molecule encoding an antibody of the present invention, including, for example, site-directed mutagenesis and PCR-mediated mutagenesis which results in amino acid substitutions. In one aspect, the derivatives include less than 10 amino acid substitutions, less than 5 amino acid substitutions, less than 4 amino acid substitutions, less than 3 amino acid substitutions, or less than 2 amino acid substitutions relative to the antibodies whose CDRs are shown in Tables 1 and 2 and/or whose heavy and light chains are shown in Figures 1-4.

In one aspect, the derivatives have conservative amino acid substitutions are made at one or more predicted non-essential amino acid residues (*i.e.,* amino acid residues which are not critical for the antibody to immunospecifically bind to the HIV protein (*e.g*., p24, p26)). A "conservative amino acid substitution" is one in which the amino acid residue is replaced with the amino acid residue having a side chain with a similar charge. Families of amino acid residues having side chains with similar charges have been defined in the art. These families include amino acids with basic side chains (*e.g*., lysine, arginine, histidine), acidic side chains (*e.g.,* aspartic acid, glutamic acid), uncharged polar side chains (*e.g.,* glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g.,* threonine, valine, isoleucine) and aromatic side chains (*e.g.,* tyrosine, phenylalanine, tryptophan, histidine). Alternatively, mutations can be introduced randomly along all or part of the coding sequence, such as by saturation mutagenesis, and the resultant mutants can be screened for biological activity to identify mutants that exhibit enhanced binding affinity to p24 or p24 fragment and/or to p26 or p26 fragment. Following mutagenesis, the encoded antibody can be expressed and the activity of the antibody can be determined.

### A) Identification of other antibodies binding the same epitope(s) as 115B-151-423 AM1 and/or 115B-151-423 AM2.

The antibodies of this disclosure need not be limited to the use of the particular antibodies whose CDRs are enumerated in Tables 1-2 and/or whose sequence shown in Figures 1-4 and/or substitutions therein as described above. In effect, each of these antibodies identifies a target epitope on HIV-1 and/or HIV-2 core proteins (p24 and p26) and these antibodies can readily be used to identify other antibodies that bind to the same epitopes with higher affinity. Thus, in certain aspects the antibodies of this invention comprise one or more antibodies that specifically bind an epitope specifically bound by an antibody of whose CDRs are enumerated in Tables 1-2 and/or whose sequence shown in Figures 1-4 (*e.g.,* 115B-151-423 AM1 and/or 115B-151-423 AM2).

Such antibodies are readily identified by screening whole antibodies, antibody fragments, or single chain antibodies for their ability to compete with the antibodies listed described above for their ability to bind to protein(s) comprising the target epitope. In other words, candidate antibodies can be screened for cross-reactivity with the antibodies 115B-151-423 AM1 and/or 115B-151-423 AM2 against the target HIV core protein(s). Means of assaying for cross-reactivity are well known to those of skill in the art (see, *e.g.,* Dowbenko et al. (1988) J. Virol. 62: 4703-4711).

For example, in certain aspects cross-reactivity can be ascertained by providing an HIV core protein (or fragment comprising the desired epitope) attached to a solid support and assaying the ability of a test antibody to compete with 115B-151-423 AM1 and/or 115B-151-423 AM2 for binding to the target protein. Thus, immunoassays in a competitive binding format are can be used for cross-reactivity determinations. For example, in one embodiment, the HIV core protein(s) or fragments thereof are immobilized to a solid support. Antibodies to be tested (*e.g*., generated by selection from a phage-display library, or generated in a whole antibody library) are added to the assay compete with one or more of the antibodies listed antibodies whose CDRs are enumerated in Tables 1-2 and/or whose sequence shown in Figures 1-4 for binding to the immobilized polypeptide. The ability of test antibodies to compete with the binding of these "prototype" antibodies to the immobilized protein are compared. The percent cross-reactivity above proteins can then calculated, using standard calculations. If the test antibody competes with one or more of the "prototype" antibodies with a comparable or greater binding affinity then the antibody is well suited for use in the present invention.

In one illustrative aspect, cross-reactivity is performed by using surface plasmon resonance in a BIAcore. In a BIAcore flow cell, the p24 and/or p26 protein(s) or fragments thereof are coupled to a sensor chip. With a typical flow rate of 5 ml/min, a titration of 100 nM to 1 µM antibody is injected over the flow cell surface for about 5 minutes to determine an antibody concentration that results in near saturation of the surface. Epitope mapping or cross-reactivity is then evaluated using pairs of antibodies at concentrations resulting in near saturation and at least 100 RU of antibody bound. The amount of antibody bound is determined for each member of a pair, and then the two antibodies are mixed together to give a final concentration equal to the concentration used for measurements of the individual antibodies. Antibodies recognizing different epitopes show an essentially additive increase in the RU bound when injected together, while antibodies recognizing identical epitopes show only a minimal increase in RU. In a particularly preferred aspect antibodies are said to be cross-reactive if, when "injected" together they show an essentially additive increase (preferably an increase by at least a factor of about 1.4, more preferably an increase by at least a factor of about 1.6, and most preferably an increase by at least a factor of about 1.8 or 2, or 4, or 8, or 10.

Cross-reactivity at the epitopes recognized by the antibodies listed described herein (*e.g.,* 115B-151-423 AM1 and/or 115B-151-423 AM2) can ascertained by a number of other standard techniques (*see, e.g.,* Geysen et al. (1987) J. Immunol. Meth. 102: 259-274).

In addition, the 115B-151-423 AM1 and/or 115B-151-423 AM2 antibodies have been sequenced (*see, e.g.,* Tables 1 and 2 and/or the Figures 1-4). The amino acid sequences comprising the complementarity determining regions (CDRs) are therefore known. Using this sequence information, the same or similar complementarity determining regions can be engineered into other antibodies to produce chimeric full size antibodies and/or antibody fragments, *e.g*., to ensure species compatibility, to increase serum half-life, and the like. A large number of methods of generating chimeric antibodies are well known to those of skill in the art (see, *e.g.,* U.S. Patent Nos: 5,502,167, 5,500,362, 5,491,088, 5,482,856, 5,472,693, 5,354,847, 5,292,867, 5,231,026, 5,204,244, 5,202,238, 5,169,939, 5,081,235, 5,075,431, and 4,975,369).

### B) Phage display methods to select other "related" anti-HIV antibodies.

### 1) Chain shuffling methods.

One approach to creating modified single-chain antibody (scFv) gene repertoires has been to replace the original V_{H} or V_{L} gene with a repertoire of V-genes to create new partners (chain shuffling) (Clackson et al. (1991) Nature. 352: 624-628). Using chain shuffling and phage display, the affinity of a human scFv antibody fragment that bound the hapten phenyloxazolone (phOx) was increased from 300 nM to 1 nM (300 fold) (Marks et al. (1992) Bio/Technology 10: 779-783).

Thus, for example, to alter the affinity of an anti-HIV antibody (*e.g*., 115B-151-423 AM1 and/or 115B-151-423 AM2, *etc.*), a mutant scFv gene repertoire is created containing a V_{H} gene comprising CDR1 (H1), and/or CDR2 (H2), and/or CDR3 (H3) of the 115B-151-423 AM1 and/or 115B-151-423 AM2 antibodies and a human V_{L} gene repertoire (light chain shuffling). The scFv gene repertoire can be cloned into a phage display vector, *e.g.,* pHEN-1 (Hoogenboom et al. (1991) Nucleic Acids Res., 19: 4133-4137) or other vectors and, after transformation, a library of transformants is obtained. This library can then be screened against HIV-1 and/or HIV-2 core protein targets to identify members with the desired binding affinity. High affinity binders can be selected and sequenced as desired.

Similarly, for heavy chain shuffling, a mutant scFv gene repertoire is created containing a V_{L} gene comprising CDR1 (L1), and/or CDR2 (L2), and/or CDR3 (L3) of the 115B-151-423 AM1 and/or 115B-151-423 AM2 antibodies and a human V_{H} gene repertoire. The scFv gene repertoire can be cloned into a phage display vector, *e.g*., pHEN-1 (Hoogenboom et al. (1991) Nucleic Acids Res., 19: 4133-4137) or other vectors and, after transformation, a library of transformants is obtained. This library can then be screened against HIV-1 and/or HIV-2 core protein targets to identify members with the desired binding affinity. High affinity binders can be selected and sequenced as desired. For detailed descriptions of chain shuffling to increase antibody affinity see Schier et al. (1996) J. Mol. Biol., 255: 28-43, and the like.

### 2) Site-directed mutagenesis to improve binding affinity.

The majority of antigen contacting amino acid side chains are typically located in the complementarity determining regions (CDRs), three in the V_{H} (CDR1 (H1), CDR2 (H2), and CDR3 (H3)) and three in the V_{L} (CDR1 (L1), CDR2 (L2), and CDR3 (L3)) (Chothia et al. (1987) J. Mol. Biol., 196: 901-917; Chothia et al. (1986) Science, 233: 755-758; Nhan et al. (1991) J. Mol. Biol., 217: 133-151). These residues typically contribute the majority of binding energetics responsible for antibody affinity for antigen. In other molecules, mutating amino acids which contact ligand has been shown to be an effective means of increasing the affinity of one protein molecule for its binding partner (Lowman et al. (1993) J. Mol. Biol., 234: 564-578; Wells (1990) Biochemistry, 29: 8509-8516). Site-directed mutagenesis of CDRs and screening against HIV-1 and/or HIV-2 core protein(s) can produce antibodies having improved binding affinity.

### 3) CDR randomization to produce higher affinity human scFv.

In an extension of simple site-directed mutagenesis, mutant antibody libraries can be created where partial or entire CDRs are randomized (V_{L} CDR1 (L1), CDR2 (L2), and/or CDR3 (L3), and/or V_{H} CDR1 (H1), CDR2 (H2), and/or CDR3 (H3)). In one embodiment, each CDR is randomized in a separate library, using a known antibody (*e.g*., 115B-151-423 AM1 and/or 115B-151-423 AM2) as a template. The CDR sequences of the highest affinity mutants from each CDR library are combined to obtain an additive increase in affinity. A similar approach has been used to increase the affinity of human growth hormone (hGH) for the growth hormone receptor over 1500 fold from 3.4 x 10⁻¹⁰ to 9.0 x 10⁻¹³ M (Lowman et al. (1993) J. Mol. Biol., 234: 564-578).

V_{H} CDR3 often occupies the center of the binding pocket, and thus mutations in this region are likely to result in an increase in affinity (Clackson et al. (1995) Science, 267: 383-386). In one embodiment, four V_{H} CDR3 residues are randomized at a time using the nucleotides NNS (*see, e.g.,* Schier et al. (1996) Gene, 169: 147-155; Schier and Marks (1996) Human Antibodies and Hybridomas. 7: 97-105, 1996; and Schier et al. (1996) J. Mol. Biol. 263: 551-567).

For example, to further increase the binding affinity the VH and VL segments of the 115B-151-423 AM1 and/or 115B-151-423 AM2 can be randomly mutated, in some embodiments preferably within the CDR2 region of VH, and/or the CDR1 region and/or CDR2 region of V_{L} in a process analogous to the *in vivo* somatic mutation process responsible for affinity maturation of antibodies during a natural immune response. This *in vitro* affinity maturation can be accomplished by replacing a portion of each CDR with a degenerate single-stranded oligonucleotide encoding three amino acids within the CDR being targeted. The replacement of a portion of each CDR with a new randomized sequence can be accomplished by homologous recombination in yeast. These randomly mutated VH and VL segments can be analyzed for binding to p24 and/or p26 fragment in the context of an scFv. Preferred scFvs exhibit an affinity to the epitope of interest with at least about a two fold improvement, at least about a three fold improvement, at least about a five fold improvement, at least about a ten fold improvement, at least about a fifteen fold improvement, at least about a twenty fold improvement, at least about a twenty-five fold improvement, at least about a thirty fold improvement, at least about a thirty-five fold improvement, at least about a forty fold improvement, at least about a forty-five fold improvement, at least about a fifty fold improvement, at least about a fifty-five fold improvement, at least about a sixty fold improvement, at least about a seventy fold improvement or at least about a seventy-five fold improvement in its equilibrium dissociation constant (K_{D}) when compared with the 115B-151-423 antibody produced by hybridoma cell line PTA-2809 (as described in U.S. Patent 7,531,640).

### C) Creation of other antibody forms.

Using the known and/or identified sequences (*e.g.* V_{H} and/or V_{L} sequences) of the antibodies provided herein other antibody forms can readily be created. Such forms include, but are not limited to multivalent antibodies, full antibodies, scFv, (scFv')₂, Fab, (Fab')₂, chimeric antibodies, and the like.

### 1) Creation of homodimers.

For example, to create (scFv')₂ antibodies, two scFvs are joined, either directly, or through a linker (*e.g.,* a carbon linker, a peptide, *etc.*), or through a disulfide bond between, for example, two cysteins. Thus, for example, to create disulfide linked scFv, a cysteine residue can be introduced by site directed mutagenesis at the carboxy-terminus of the antibodies described herein.

An scFv can be expressed from this construct, purified by IMAC, and analyzed by gel filtration. To produce (scFv')₂ dimers, the cysteine is reduced by incubation with 1 mM 3-mercaptoethanol, and half of the scFv blocked by the addition of DTNB. Blocked and unblocked scFvs are incubated together to form (scFv')₂ and the resulting material can be analyzed by gel filtration. The affinity of the resulting dimer can be determined using standard methods, *e.g.* by BIAcore.

In one illustrative aspect, the (scFv')₂ dimer is created by joining the scFv' fragments through a linker, *e.g.,* a peptide linker. This can be accomplished by a wide variety of means well known to those of skill in the art. For example, one approach is described by Holliger et al. (1993) Proc. Natl. Acad. Sci. USA, 90: 6444-6448 (*see also* WO 1994/013804).

It is noted that using the V_{H} and/or V_{L} sequences provided herein Fabs and (Fab')₂ dimers can also readily be prepared. Fab is a light chain joined to V_{H}-C_{H}1 by a disulfide bond and can readily be created using standard methods known to those of skill in the art. The F(ab)'₂ can be produced by dimerizing the Fab, *e.g.* as described above for the (scFv')₂ dimer.

### 2) Chimeric antibodies.

The antibodies of this invention also include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (*see, e.g.,* U.S. Pat. No. 4,816,567; Morrison et al. (1984) Proc. Natl. Acad. Sci. 81: 6851-6855, *etc.*).

Chimeric antibodies are antibodies comprising a portions from two different species (*e.g.* a human and non-human portion). Typically, the antigen combining region (or variable region) of a chimeric antibody is derived from a one species source and the constant region of the chimeric antibody (which confers biological effector function to the immunoglobulin) is derived from another source. A large number of methods of generating chimeric antibodies are well known to those of skill in the art (*see, e.g.,* U.S. Patent Nos: 5,502,167, 5,500,362, 5,491,088, 5,482,856, 5,472,693, 5,354,847, 5,292,867, 5,231,026, 5,204,244, 5,202,238, 5,169,939, 5,081,235, 5,075,431, and 4,975,369.

In general, the procedures used to produce chimeric antibodies consist of the following steps (the order of some steps may be interchanged): (a) identifying and cloning the correct gene segment encoding the antigen binding portion of the antibody molecule; this gene segment (known as the VDJ, variable, diversity and joining regions for heavy chains or VJ, variable, joining regions for light chains, or simply as the V or variable region or V_{H} and V_{L} regions) may be in either the cDNA or genomic form; (b) cloning the gene segments encoding the human constant region or desired part thereof; (c) ligating the variable region to the constant region so that the complete chimeric antibody is encoded in a transcribable and translatable form; (d) ligating this construct into a vector containing a selectable marker and gene control regions such as promoters, enhancers and poly(A) addition signals; (e) amplifying this construct in a host cell (*e.g*., bacteria); (f) introducing the DNA into eukaryotic cells (transfection) most often mammalian lymphocytes; and culturing the host cell under conditions suitable for expression of the chimeric antibody.

Antibodies of several distinct antigen binding specificities have been manipulated by these protocols to produce chimeric proteins (*e.g.,* anti-TNP: Boulianne et al. (1984) Nature, 312: 643; and anti-tumor antigens: Sahagan et al. (1986) J. Immunol., 137: 1066). Likewise several different effector functions have been achieved by linking new sequences to those encoding the antigen binding region. Some of these include enzymes (Neuberger et al. (1984) Nature 312: 604), immunoglobulin constant regions from another species and constant regions of another immunoglobulin chain (Sharon et al. (1984) Nature 309: 364; Tan et al., (1985) J. Immunol. 135: 3565-3567).

### 3) Intact human antibodies.

In another aspect, this invention discloses intact, fully human (or fully non-human) antibodies. Such antibodies can readily be produced in a manner analogous to making chimeric human antibodies. In this instance, instead of using a recognition function derived, *e.g.* from a murine, the fully human recognition function (*e.g.,* V_{H} and V_{L}) of the antibodies described herein is utilized.

### 4) Diabodies.

In certain aspects this invention contemplates diabodies comprising V_{H} and/or V_{L} domains comprising one or more of the CDRs described herein. In certain aspects the V_{H} and/or V_{L} domains comprise all three (CDR1, CDR2, CDR3) of the CDRs comprising the V_{H} and/or V_{L} domains of 115B-151-423 AM1 and/or 115B-151-423 AM2. The term "diabodies" refers to antibody fragments typically having two antigen-binding sites. The fragments typically comprise a heavy chain variable domain (V_{H}) connected to a light chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161, and Holliger et al. (1993) Proc. Natl. Acad. Sci. USA 90: 6444-6448.

### 5) Unibodies

In certain aspects the anti-HIV antibodies are engineered as unibodies. Unibodies provide an antibody technology that produces a stable, smaller antibody format with an anticipated longer therapeutic window than certain small antibody formats. In certain aspects unibodies are produced from IgG4 antibodies by eliminating the hinge region of the antibody. Unlike the full size IgG4 antibody, the half molecule fragment is very stable and is termed a unibody. Halving the IgG4 molecule leaves only one area on the unibody that can bind to a target. Methods of producing unibodies are described in detail in PCT Publication WO 2007/059782, which is incorporated herein by reference in its entirety (*see, also,* Kolfschoten et al. (2007) Science 317: 1554-1557).

In certain aspects a randomized unibody library is generated and screened for binding specificity and affinity for p24 and/or p26 comparable to or greater than 115B-151-423 AM1 and/or 115B-151-423 AM2. In certain aspects the CDRs identified above are engineered into a unibody framework/structure.

### 6) Affibodies.

In certain aspects the anti-HIV antibodies described herein are engineered as affibodies. Affibody molecules are class of affinity proteins based on a 58-amino acid residue protein domain, derived from one of the IgG-binding domains of staphylococcal protein A. This three helix bundle domain has been used as a scaffold for the construction of combinatorial phagemid libraries, from which Affibody variants that target the desired molecules can be selected using phage display technology (*see, e.g.,.* Nord et al. (1997) Nat. Biotechnol. 15: 772-777; Ronmark et al. (2002) Eur. J. Biochem., 269: 2647-2655.). Details of Affibodies and methods of production are known to those of skill (*see, e.g.,* US Patent No 5,831,012).

In certain aspects a randomized affibody library is generated and screened for binding specificity and affinity for p24 and/or p26 comparable to or greater than 115B-151-423 AM1 and/or 115B-151-423 AM2. In certain aspects the CDRs identified above are engineered into an affibody structure.

### 7) Nanobodies.

In certain aspects the HIV antibodies described herein can be engineered as nanobodies (VₕH antibodies). Methods of making VₕH (nanobodies) are also well known to those of skill in the art. The Camelidae heavy chain antibodies are found as homodimers of a single heavy chain, dimerized via their constant regions. The variable domains of these camelidae heavy chain antibodies are referred to as V_{HH} domains or V_{HH}, and can be either used *per se* as nanobodies and/or as a starting point for obtaining nanobodies. Isolated V_{HH} retain the ability to bind antigen with high specificity (*see, e.g.,* Hamers-Casterman et al. (1993) Nature 363: 446-448). In certain aspects such V_{HH} domains, or nucleotide sequences encoding them, can be derived from antibodies raised in Camelidae species, for example in camel, dromedary, llama, alpaca and guanaco. Other species besides Camelidae (*e.g.* shark, pufferfish) can produce functional antigen-binding heavy chain antibodies, from which (nucleotide sequences encoding) such naturally occurring V_{HH} can be obtained, *e.g.* using the methods described in U.S. Patent Publication US 2006/0211088.

In certain aspects a randomized nanobody library is generated and screened for binding specificity and affinity for p24 and/or p26 comparable to or greater than 115B-151-423 AM1 and/or 115B-151-423 AM2. In certain aspects the CDRs identified above are engineered into an nanobody structure.

In short, using routine methods, the antibodies whose CDRs are enumerated in Tables 1-2 and/or whose sequence shown in Figures 1-4 can readily be used to generate or identify other antibodies (full length, antibody fragments, single-chain, and the like) that bind to the same epitope. Similarly, these can readily be utilized to generate other antibodies that have the same or similar complementarity determining regions (CDRs), but provide greater specificity and/or affinity to their p24 and/or p26 epitope(s).

### II. Nucleic acids encoding anti-HIV antibodies.

In one aspect, the isolated nucleic acid molecules are provided that encode any of the antibodies described above. Thus, for example, disclosed is an isolated nucleic acid molecule encoding an antibody that binds to an a p26 and/or a p24 epitope with at least about a two fold improvement, at least about a three fold improvement, at least about a four fold improvement, at least about a five fold improvement, at least about a six fold improvement, at least about a seven fold improvement, at least about an eight fold improvement, at least about a nine fold improvement, at least about a ten fold improvement, at least about an eleven fold improvement, at least about a twelve fold improvement, at least about a thirteen fold improvement, at least about a fourteen fold improvement, at least about a fifteen fold improvement, or at least about a twenty fold improvement in its equilibrium dissociation constant (K_{D}) when compared with the HIV 115B-151-423 mAb (PTA-2809 cell line) described in U.S. Patent 7,531,640 (*see, e.g.,* col. 8) and deposited with the American Type Culture Collection, 10801 University Boulevard Manassas, VA 20110 under the term s of the Budapest treaty on December 4, 2001).

Also disclosed is an isolated nucleic acid molecule that comprises a nucleotide sequence that hybridizes, under stringent conditions, to the nucleic acid molecule described herein that encodes an antibody that binds to an a p26 and/or a p24 epitope with at least about a two fold improvement, at least about a three fold improvement, at least about a four fold improvement, at least about a five fold improvement, at least about a six fold improvement, at least about a seven fold improvement, at least about an eight fold improvement, at least about a nine fold improvement, at least about a ten fold improvement, at least about an eleven fold improvement, at least about a twelve fold improvement, at least about a thirteen fold improvement, at least about a fourteen fold improvement, at least about a fifteen fold improvement, or at least about a twenty fold improvement in its equilibrium dissociation constant (K_{D}) when compared with the HIV 115B-151-423 mAb described above.

In certain aspects the nucleic acid encodes an antibody that immunospecifically binds to p24 and/or to p26 with an equilibrium dissociation constant (KD) of less than about 10 pM, more preferably less than about 9 pM, still more preferably less than about 8 pM, and in certain embodiments, with a KD between about 7 pM and about 100 fM.

Accordingly, in certain aspects, isolated nucleic acids are contemplated that encode an antibody that comprises a light chain variable domain comprising complementarity determining regions L1, L2 and L3, and a heavy chain variable domain comprising complementarity determining regions HI, H2 and H3 wherein: the amino acid sequences of one or more of the L1, L2, or L3 CDRs of the light chain variable domain comprise the amino acid sequences respectively of the L1, L2, and/or L3 CDRs of an antibody selected from the group consisting of 115B-151-423 AM1 and 115B-151-423 AM2; and/or the amino acid sequences of one or more of the H1, H2, or H3 CDRs of the heavy chain variable domain comprise the amino acid sequences respectively of the H1, H2, and/or H3 CDRs of an antibody selected from the group consisting of 115B-151-423 AM1 and 115B-151-423 AM2. In certain aspects the L3 CDR of the antibody comprises the amino acid sequence of the L3 CDR of an antibody selected from the group consisting of 115B-151-423 AM1 and 115B-151-423 AM2; and/or the H2 and/or H3 CDRs of the antibody comprise the amino acid sequences respectively of the H2 and H3 CDRs of an antibody selected from the group consisting of 115B-151-423 AM1 and 115B-151-423 AM2.

In certain aspects nucleic acid encodes an antibody where the H2 and H3 CDRs of the antibody comprise the amino acid sequences respectively of the H2 and H3 CDRs of an antibody selected from the group consisting of 115B-151-423 AM1 and 115B-151-423 AM2. In certain aspects the H1, H2 and H3 CDRs of the antibody comprise the amino acid sequences respectively of the H1, H2 and H3 CDRs of an antibody selected from the group consisting of 115B-151-423 AM1 and 115B-151-423 AM2; and/or the L1, L2 and L3 CDRs of the antibody comprise the amino acid sequences respectively of the L1, L2 and L3 CDRs of an antibody selected from the group consisting of 115B-151-423 AM1 and 115B-151-423 AM2.

In certain aspects nucleic acid encodes an antibody where H1, H2 and H3 CDRs of the antibody comprise the amino acid sequences respectively of the H1, H2 and H3 CDRs of an antibody selected from the group consisting of 115B-151-423 AM1 and 115B-151-423 AM2.

In certain aspects nucleic acid encodes an antibody where the L1, L2 and L3 CDRs of the antibody comprise the amino acid sequences respectively of the L1, L2 and L3 CDRs of an antibody selected from the group consisting of 115B-151-423 AM1 and 115B-151-423 AM2.

In certain aspects nucleic acid encodes an antibody where the H1, H2 and H3 CDRs of the antibody comprise the amino acid sequences respectively of the H1, H2 and H3 CDRs of 115B-151-423 AM1; and the L1, L2 and L3 CDRs of the antibody comprise the amino acid sequences respectively of the L1, L2 and L3 CDRs of 115B-151-423 AM1.

In certain aspects nucleic acid encodes an antibody where the H1, H2 and H3 CDRs of the antibody comprise the amino acid sequences respectively of the H1, H2 and H3 CDRs of 115B-151-423 AM2; and the L1, L2 and L3 CDRs of the antibody comprise the amino acid sequences respectively of the L1, L2 and L3 CDRs of 115B-151-423 AM2.

In certain aspects the nucleic acid encodes an antibody as shown in Figures 1-4.

In certain aspects the nucleic acid encode antibodies The derived or variant antibodies of the present invention comprises at least one mutation (such as deletions, additions and/or substitutions) in at least one of the heavy chain complementary determining ("CDR") regions (for example, the heavy chain CDR 1, heavy chain CDR 2, or heavy chain CDR 3), at least one mutation (such as deletions, additions and/or substitutions) in the light chain CDR regions (for example, the light chain CDR 1, light chain CDR 2, or light chain CDR 3) as described above.

### III. Preparation of Antibody Molecules.

The antibodies described herein (*e.g.,* full size antibodies, bispecific antibodies, polyspecific antibodies, single chain antibodies, unibodies, nanobodies, affibodies, single chain antibodies, chimeric antibodies, and immunospecific binding fragments thereof) described herein can be made by methods well known to those of skill in the art.

### A) Chemical synthesis.

Using the sequence information provided herein, for example, the antibodies of this invention (*e.g*., 115B-151-423 AM1 and/or 115B-151-423 AM2, *etc.),* or variants thereof, can be chemically synthesized using well known methods of peptide synthesis. Solid phase synthesis in which the C-terminal amino acid of the sequence is attached to an insoluble support followed by sequential addition of the remaining amino acids in the sequence is one preferred method for the chemical synthesis of single chain antibodies. Techniques for solid phase synthesis are described by Barany and Merrifield, Solid Phase Peptide Synthesis; pp. 3-284 in The Peptides: Analysis, Synthesis, Biology. Vol. 2: Special Methods in Peptide Synthesis, Part A., Merrifield et al. (1963) J. Am. Chem. Soc., 85: 2149-2156, and Stewart et al. (1984) Solid Phase Peptide Synthesis, 2nd ed. Pierce Chem. Co., Rockford, Ill.

### B) Recombinant expression of antibodies.

The antibodies of this invention (*e.g*., 115B-151-423 AM1 and/or 115B-151-423 AM2, *etc.*), or variants thereof, are prepared using standard techniques well known to those of skill in the art. Using the sequence information provided herein, nucleic acids encoding the desired antibody can be chemically synthesized according to a number of standard methods known to those of skill in the art. Oligonucleotide synthesis, is preferably carried out on commercially available solid phase oligonucleotide synthesis machines (Needham-VanDevanter et al. (1984) Nucleic Acids Res. 12: 6159-6168) or manually synthesized using the solid phase phosphoramidite triester method described by Beaucage *et. al.* (Beaucage et. al. (1981) Tetrahedron Letts. 22(20): 1859-1862).

Alternatively, nucleic acids encoding the antibody can be amplified and/or cloned according to standard methods. Thus, the antibodies described herein can be prepared by recombinant expression of, for example, nucleic acids encoding light and heavy chains in a host cell. To express the antibody recombinantly, the host cell is transfected with one or more recombinant expression vectors carrying nucleic acid molecules encoding the antibody such that the expressed antibody protein is expressed in the host cell and, preferably, secreted into the medium in which the host cells is cultured, from which medium the antibody can be recovered. Molecular cloning techniques to achieve these ends are known in the art. A wide variety of cloning and *in vitro* amplification methods are suitable for the construction of recombinant nucleic acids. Examples of these techniques and instructions sufficient to direct persons of skill through many cloning exercises are found in Berger and Kimmel, Guide to Molecular Cloning Techniques, Methods in Enzymology volume 152 Academic Press, Inc., San Diego, CA (Berger); Sambrook et al. (1989) Molecular Cloning - A Laboratory Manual (2nd ed.) Vol. 1-3, Cold Spring Harbor Laboratory, Cold Spring Harbor Press, NY, (Sambrook); and Current Protocols in Molecular Biology, F.M. Ausubel et al., eds., Current Protocols, a joint venture between Greene Publishing Associates, Inc. and John Wiley & Sons, Inc., (1994 Supplement) (Ausubel). Methods of producing recombinant immunoglobulins are also known in the art. *See,* Cabilly, U.S. Patent No. 4,816,567; and Queen et al. (1989) Proc. Natl Acad. Sci. USA 86: 10029-10033. In addition, detailed protocols for the expression of antibodies are also provided by Liu et al. (2004) Cancer Res. 64: 704-710, Poul et al. (2000) J. Mol. Biol. 301: 1149-1161, and the like.

To express the antibodies of the invention, nucleic acid molecule(s) encoding the antibody domain(s) (*e.g*., the light and heavy chain regions) are first obtained. These nucleic acid molecules may be obtained from a hybridoma cell line expressing a monoclonal antibody 115B-151-423 (described in U.S. Patent 7,531,640) and modified by means well known in the art (such as site-directed mutagenesis) to generate antibodies of the present invention.

In various aspects the nucleic acid sequence(s) encoding the antibody can be chemically synthesized or obtained PCR amplification and/or recombinant expression according to standard methods well known to those of skill in the art.

Once the nucleic acids encoding the antibody are obtained, these nucleic acid fragments can be further manipulated by standard recombinant DNA techniques, for example to convert the variable region genes to an antibody (such as, but not limited to, a full-length antibody chain genes, to Fab fragment genes or to a scFv gene). In these manipulations, a VL- or VH-encoding nucleic acid fragment is operatively linked to another nucleic acid fragment encoding another protein, such as antibody constant region or a flexible linker. The term "operatively linked", as used in this context, is intended to mean that the two nucleic acid fragments are joined such that the amino acid sequences encoded by the two nucleic acid fragments remain in-frame.

In an alternative method, an scFv gene may be constructed with wild type CDR regions (such as those of monoclonal antibody 115B-151-423) and then mutated using techniques known in the art.

The isolated nucleic acid molecule encoding the VH region can be converted to a full-length heavy chain gene by operatively linking the VH-encoding nucleic acid molecule to another nucleic acid molecule encoding heavy chain constant regions (CHI, CH2 and CH3). The sequences of human heavy chain constant region genes are known in the art (See for example, Kabat et al. (1991) Sequences of Proteins of Immunological Interest, Fifth Edition, U.S. Department of Health and Human Services, NIH Publication No. 91-3242). In another aspect, antibodies described herein include all known human heavy chain constant regions, including but not limited to, all known allotypes of the human heavy chain constant region. Nucleic acid fragments encompassing these regions can be obtained by standard PCR amplification. The heavy chain constant region can, for example, be an IgG1, IgG2, IgG3, IgG4, IgA, IgE, IgM or IgD constant region.

The isolated nucleic acid molecule encoding the VL region can be converted to a full-length light chain gene (as well as a Fab light chain gene) by operatively linking the VL-encoding nucleic acid molecule to another nucleic acid molecule encoding the light chain constant region, CL. The sequences of human light chain constant region genes are known in the art (see *e.g.,* Kabat *et al. supra*). In various aspects the antibodies described herein include all known human light chain constant regions, including but not limited to, all known allotypes of the human light chain constant region. Nucleic acid fragments encompassing these regions can be obtained by standard PCR amplification. The light chain constant region can be a kappa or lambda constant region, but most preferably is a kappa constant region.

It is to be understood that the specific designations of framework (FR) and CDR regions within a particular heavy or light chain region may vary depending on the convention or numbering system used to identify such regions (*e.g.* Chothia, Kabat, Oxford Molecular's *AbM* modeling software, *etc.,* all of which are known to those of ordinary skill in the art). For the purposes of the present description, the Kabat numbering system is used.

To create a scFv gene, the VH- and VL-encoding nucleic acid fragments are operatively linked to directly to each other or to each other via nucleic acid encoding a flexible linker. Illustrative linkers are shown above in Table 3 and include but are not limited to, for example the (Gly4Ser) (SEQ ID NO:24) linker, the GPAKELTPLKEAKVS (SEQ ID NO:26) linker (see, U.S. Patent Publication 2004-0175379 A1), and the like. Examples of other linker sequences that can be used are well known to those of skill the art *(see, e.g.,* Bird et al. (1988) Science 242:423-426; Huston et a/. (1988) Proc. Natl. Acad. Sci., USA, 85: 5879-5883; McCafferty et al. (1990) Nature, 348:552-554; Huston et al. (1991) Meth. Enzymol., 203: 46-88; Johnson and Bird (1991) Meth. Enzymol., 203: 88-89; and the like).

To express the antibodies, or antibody portions, nucleic acid molecules encoding partial or full-length antibody regions obtained as described above, are inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vectors or, more typically, both genes are inserted into the same expression vector. The antibody genes are inserted into the expression vector by standard methods (for example, ligation of complementary restriction sites on the antibody gene fragment and vector, or blunt end ligation if no restriction sites are present). Prior to the insertion of the light or heavy chain sequences, the expression vector may already carry antibody constant region sequences. For example, one approach to converting the VH and VL sequences to full-length antibody genes is to insert them into expression vectors already encoding heavy chain constant and light chain constant regions, respectively, such that the VH segment is operatively linked to the CH "segment" within the vector and the VL segment is operatively linked to the CL segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. In certain embodiments the single peptide can be an immunoglobin signal peptide or a heterologous signal peptide (*e.g.,* a signal peptide from a non-immunoglobulin protein).

In addition to the antibody chain genes, the recombinant expression vectors can carry regulatory sequences that control the expression of the antibody chain genes in a host cell. The term "regulatory sequence" is intended to include promoters, enhancers and other expression control elements (*e.g*., polyadenylation signals) that control the transcription or translation of the antibody chain genes. Such regulatory sequences are described, for example, in Goeddel (1990) Gene Expression Technology. Methods in Enzymology 185, Academic Press, San Diego, Calif. It will be appreciated by those skilled in the art that the design of the expression vector, including the selection of regulatory sequences may depend on such factors as the choice of the host cell to be transformed, the level of the expression of protein desired, *etc.* Certain preferred regulatory sequences for mammalian host cell expression can include viral elements that direct high levels of protein expression in mammalian cells, such as promoters and/or enhancers derived from cytomegalovirus ("CMV") (such as the CMV promoter/enhancer), Simian Virus 40 ("SV40") (such as the SV40 promoter/enhancer), adenovirus, (such as the adenovirus major late promoter ("AdMLP")) and polyoma. For further description of viral regulatory elements, and sequences thereof, see for example, U.S. Patent Nos 5,168,062, 4,510,245, and 4,968,615.

In addition to the antibody chain genes and regulatory sequences, recombinant expression vectors may carry additional sequences, such as sequences that regulate replication of the vector in host cells (*e.g*., origins of replication) and selectable marker genes. The selectable marker gene facilitates selection of host cells into which the vector has been introduced (*see, e.g.,* U.S. Patent Nos. 4,399,216, 4,634,665 and 5,179,017). For example, typically the selectable marker gene confers resistance to drugs, such as G418, hygromycin or methotrexate, on a host cell into which the vector has been introduced. Illustrative selectable marker genes include, but are not limited to the dihydrofolate reductase ("DHFR") gene for use in dhfr-host cells with methotrexate selection/amplification and the neomycin ("neo") gene for G418 selection.

For expression of the light and heavy chains, the expression vector(s) encoding the heavy and light chains are transfected into a host cell by standard techniques. The various forms of the term "transfection" are intended to encompass a wide variety of techniques commonly used for the introduction of exogenous DNA into a prokaryotic or eukaryotic host cell, *e.g*., electroporation, calcium-phosphate precipitation, DEAE-dextran transfection and the like. Although it is theoretically possible to express the antibodies of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active intact antibody. Prokaryotic expression of antibody genes has been reported to be ineffective for production of high yields of active antibody (See, Boss, M. A. and Wood, C. R, Immunology Today 6:12-13 (1985)).

Mammalian host cells suitable for expressing the recombinant antibodies of the invention include, but are not limited to, the Chinese Hamster Ovary ("CHO") cells (including dhfr-CHO cells, described in Urlaub and Chasin (1980) Proc. Natl. Acad. Sci., USA, 77: 4216-4220, used with a DHFR selectable marker, for example, as described by Kaufman and. Sharp (1982) Mol. Biol., 159: 601-621), NSO myeloma cells, COS cells, ,and SP2/0 myeloma cells. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

Host cells can also be used to produce portions of intact antibodies, such as Fab fragments, F(ab')₂ fragments or scFv molecules. It will be understood that variations on the above procedure are within the scope of the present invention. For example, it may be desirable to transfect a host cell with nucleic acid molecule encoding either the light chain or the heavy chain (but not both) of an antibody of the present invention. Recombinant DNA technology may also be used to remove some or all of the nucleic acid molecules encoding either or both of the light and heavy chains that are not necessary for binding to the HIV protein (*e.g.,* p24 and/or p26). The molecules expressed from such truncated nucleic acid molecules also are encompassed by the antibodies of the invention.

In one illustrative system for recombinant expression of an antibody, or antigen binding portion thereof a recombinant expression vector encoding both the antibody heavy chain and the antibody light chain is introduced into dhfr-CHO cells (*e.g.,* by calcium phosphate-mediated transfection). Within the recombinant expression vector, the antibody heavy and light chain genes are each operatively linked to CMV enhancer/AdMLP promoter regulatory elements to drive high levels of transcription of the genes. The recombinant expression vector can also carry a DHFR gene, which allows for selection of CHO cells that have been transfected with the vector. Cells can be cultured in medium without hypoxanthine and thymidine to obtain those CHO cells that have acquired the DHFR gene from the transfecting vector.

Antigen specific screening methods can be used to identify those clones that express the highest quantity of antibody. Those individual clones are expanded and can be re-screened and preferred cell lines selected. The selected transformant host cells are cultured to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium.

It will also be recognized that in certain aspects phage and yeast expression systems can also be used to express and recover antibody particularly single chain antibody.

The foregoing methods are illustrative and not intended to be limiting. Using the teaching provided herein, other methods of expressing antibodies, or antibody fragments described herein will be available to one of skill in the art.

### IV. Immunoassays.

In various embodiments the antibodies described herein or antibody fragments thereof may be used in immunoassays for the detection of HIV-1 core proteins or protein fragments (Groups M and O), HIV-2, or HIV-1 and HIV-2 simultaneously in a sample. An antibody "fragment" is a subunit of the antibody that reacts in the same manner, functionally, as the full antibody with respect to binding properties (*e.g*., has substantially the same specificity and/or affinity). The immunoassays can be conducted using any format known in the art, such as, but not limited to, a sandwich format, a competitive inhibition format (including both forward or reverse competitive inhibition assays), a fluorescence polarization format, and the like.

It is believed that when the antibodies described herein are used in combination in an immunoassay, for example, in a sandwich assay, one may minimally detect core antigen (p24) from subtypes A, B, C, D, E, F, G and O of HIV-1 groups M and O, and HIV-2 core antigen (p26) in a patient sample. It is also believed that than 25 picogram (*i.e.,* picogram core antigen/ml of serum or plasma) quantities of the HIV-1 p24 antigen and HIV-2 p26 antigen can be detected using the combinations of antibodies described above.

In certain immunoassays for the qualitative detection of HIV-1 core protein (p24) and/or HIV-2 core protein (p26) or fragments thereof in a test sample, at least one anti-HIV antibody described herein is contacted with at least one test sample suspected of containing or that is known to contain HIV core protein or protein fragment to form an antibody-HIV core protein immune complex. The antibodies described in Section I herein can be used in such immunoassays to form such antibody-HIV core protein immune complexes in at least one test sample. These immune complexes can then detected using routine techniques known to those skilled in the art. For example, the anti-HIV antibody can be labeled with a detectable label to detect the presence of an antibody-HIV core protein complex. Alternatively, the HIV-1 and/or HIV-2 core proteins or fragments thereof in the test sample can be labeled with a detectable label and the resulting antibody-HIV protein immune complexes detected using routine techniques known to those skilled in the art. Detectable labels and their attachment to antibodies are discussed in more detail *infra.*

Alternatively, a second antibody that binds to the p24 and/or p26 and/or fragments thereof and that contains a detectable label can be added to the test sample and used to detect the presence of the antibody-HIV protein complex. Any detectable label known in the art can be used. Detectable labels and their attachment to antibodies are discussed in more detail *infra.*

In general, sandwich assays for the qualitative or quantitative detection of HIV-1 and/or HIV-2 core proteins (p24 and/or p26) or fragments thereof, utilizes a first antibody to capture the HIV-1 and/or HIV-2 protein or protein fragment forming an antibody/HIV protein immunocomplex which is then bound by a second antibody comprising a detectable label (*e.g.,* a signal-generating compound capable of generating a detectable signal), or a tag that captures a detectable label. Either the first (capture) antibody or the second antibody can comprise one or more anti-HIV antibodies described herein. Detection of the signal thus indicates presence of the complexes and thus presence of the antigen in the sample. The amount of antigen(s) in the test sample may also be calculated, as the signal generated is proportional to the amount of antigen in the sample (*see, e.g.,* U.S. Patent No: 6,015,662). In various embodiments the first (capture) antibody is adsorbed onto a solid or semisolid (*e.g.,* gel) phase (*e.g.,* a microparticle, a microtiter well, a bead, *etc.*), or complexed with a fluid that can be separated *e.g.,* via a partition separation. Examples of solid phases used in immunoassays include, but are not limited to are porous and non-porous materials, latex particles, magnetic particles, microparticles, beads, membranes, microtiter wells. test strips, and plastic tubes.

In one illustrative embodiment 115B-151-423 AM1 and 115B-151-423 AM2 or a fragment thereof is provided adsorbed onto a solid or gel phase. The test sample is then contacted with the antibody or fragment thereof such that, if p24 antigen and/or p26 antigen is present in the patient sample, antibody/antigen complexes are formed as a first mixture. For example, both antibody/p24 antigen and antibody/p26 antigen complexes may be formed if the subject/sample has both HIV-1 and HIV-2. One then adds a conjugate comprising a probe antibody, that binds an epitope distinct from and compatible with the epitope bound by 115B-151-423 AM1 or 115B-151-423 AM2 and a signal-generating compound. Antibody/antigen/antibody probe complexes are then formed as a second mixture. HIV-1 and/or HIV-2 antigen is then detected in the sample by detecting the presence of the signal generated and thus the antibody/antigen/antibody probe complexes.

Another manner of detecting the complexes formed is to utilize a conjugate comprising a third antibody attached to a signal-generating compound. In particular, once the antibody/antigen/antibody complexes described above have formed (*i.e.,* the latter antibody being the second antibody which is unlabelled), one may then add a conjugate which binds to the second unlabelled antibody in solution. The conjugate may comprise, for example, an antigen or anti-antibody capable of binding to the bound second antibody attached to a signal-generating compound capable of generating a detectable signal. Detection of the signal thus indicates presence of the complexes and thus presence of the antigen in the sample.

The design of the assay is dependent upon the affinities and specificities of the antibodies used, accuracy of results obtained, convenience, the nature of the solid phase, *etc.* (See U.S. Pat. No. 5,104,790 for a discussion of different antigen assay formats.) Additionally, it should also be noted that the initial capture antibody used in the immunoassay may be covalently or non-covalently (*e.g.,* ionic, hydrophobic, *etc.*) attached to the solid or gel phase. The antibody (or antibodies) can be bound to the solid support or gel support by adsorption, by covalent bonding using a chemical coupling agent or by other means known in the art, provided that such binding does not interfere with the ability of the antibody to bind the target analyte (*e.g*., HIV core protein). Moreover, if necessary, the solid support can be derivatized to allow reactivity with various functional groups on the antibody. Such derivatization can involve the use of certain coupling agents such as, but not limited to, maleic anhydride, N-hydroxysuccinimide and 1-ethyl-3-(3-dimethylaminopropyl)carbodiimide.

As noted above, in certain embodiments the conjugate (or indicator reagent) will comprise an antibody (or perhaps anti-antibody, depending upon the assay), attached to a signal-generating compound or label. This signal-generating compound or "label" is in itself detectable or may be reacted with one or more additional compounds to generate a detectable product. Examples of signal-generating compounds include chromogens, radioisotopes (*e.g*., ¹²⁵I, ¹³¹I, ³²P, ³H, ³⁵S and ¹⁴C), chemiluminescent compounds (*e.g.,* acridinium), particles (visible or fluorescent), quantum dots, nanoparticles, nucleic acids, complexing agents, or catalysts such as enzymes (*e.g*., alkaline phosphatase, acid phosphatase, horseradish peroxidase, beta-galactosidase and ribonuclease). In the case of enzyme use (*e.g*., alkaline phosphatase or horseradish peroxidase), addition of a chromo-, fluro-, or lumo-genic substrate results in generation of a detectable signal. Other detection systems such as time-resolved fluorescence, internal-reflection fluorescence, amplification (*e.g*., polymerase chain reaction) and Raman spectroscopy are also useful.

Another illustrative assay in which the antibodies described herein may be utilized involves simultaneously contacting: 1) one antibody (bound to a solid support), 2) the test sample and 3) an indicator reagent comprising an antibody or fragment thereof (*e.g.,* 115B-151-423 AM1 or 115B-151-423 AM2) to which a signal generating compound is attached, to form a mixture. The mixture is then incubated for a time and under conditions sufficient to form antibody/antigen/antibody complexes. The presence, if any, of HIV-1 and/or HIV-2 antigen present in the test sample and captured on the solid phase is determined by detecting the measurable signal generated by the signal-generating compound. The amount of antigen present in the test sample is proportional to the signal generated. In this assay or those described above, the antibodies described herein may be used either as the capture phase or as part of the indicator reagent in solution (*i.e.,* the reagent comprising an antibody and a signal-generating compound).

In a forward competitive format, an aliquot of labeled HIV-1 and/or HIV-2 core protein, or fragments thereof, of a known concentration is used to compete with p24 and/or p26 or fragment thereof in a test sample for binding to the HIV-1 antibody (such as an antibody of the present invention). Peptides of HIV-1 and/or HIV-2 core protein, or fragments thereof and methods of making such peptides are known in the art.

In a forward competition assay, an immobilized antibody (such as an anti-HIV antibody of the present invention) can either be sequentially or simultaneously contacted with the test sample and a labeled HIV-1 and/or HIV-2 core protein, or fragment thereof. The HIV-1 and/or HIV-2 core protein, or fragments thereof, can be labeled with any detectable label known to those skilled in the art, including those detectable labels discussed above in connection with the sandwich assay format. In this assay, the antibody of the present invention can be immobilized on to a solid support using the techniques discussed previously herein. Alternatively, the antibody of the present invention can be coupled to an antibody, such as an antispecies antibody, that has been immobilized on to a solid support, such as a microparticle,

The labeled HIV-1 and/or HIV-2 core protein, or fragments thereof, the test sample and the antibody are incubated under conditions similar to those described above in connection with the sandwich assay format. Two different species of antibody-HIV protein complexes are then generated. Specifically, one of the antibody-HIV protein complexes generated contains a detectable label while the other antibody-HIV protein complex does not contain a detectable label. The antibody-HIV protein complex can be, but does not have to be, separated from the remainder of the test sample prior to quantification of the detectable label. Regardless of whether the antibody-HIV protein complex is separated from the remainder of the test sample, the amount of detectable label in the antibody-HIV protein complex is then quantified. The concentration of HIV-1 and/or HIV-2 core protein, or fragments thereof, in the test sample can then be determined by comparing the quantity of detectable label in the antibody-HIV protein complex to a standard curve. The standard curve can be generated using serial dilutions of HIV-1 and/or HIV-2 core protein, or fragments thereof, by mass spectroscopy, gravimetrically and by other techniques known in the art.

The antibody-HIV protein complex can be separated from the test sample by binding the antibody to a solid support, such as the solid supports discussed above in connection with the sandwich assay format, and then removing the remainder of the test sample from contact with the solid support.

In certain aspects a reverse competition assay, using the anti-HIV antibodies described herein can also be utilized.

In certain embodiments a fluorescence polarization assay is provided. In one embodiment, an anti-HIV antibody (*e.g*., as described herein) or functionally active fragment thereof is first contacted with an unlabeled test sample suspected of containing HIV-1 and/or HIV-2 proteins or fragments thereof to form an unlabeled HIV protein-antibody complex. The unlabeled HIV protein -antibody complex is then contacted with a fluorescently labeled HIV protein, HIV protein fragment or HIV protein analogue thereof. The labeled HIV protein, HIV protein fragment or HIV protein analogue competes with any unlabeled HIV protein or HIV protein fragment in the test sample for binding to the antibody or functionally active fragment thereof. The amount of labeled HIV protein-antibody complex formed is determined and the amount of HIV-1 and/or HIV-2 protein in the test sample determined, *e.g*., via use of a standard curve.

In the various assays described herein, detection of detectable labels is by means well known to those of skill in the art. For example, if an enzymatic label is used, the labeled complex is reacted with a substrate for the label that gives a quantifiable reaction such as the development of color. If the label is a radioactive label, the label is quantified using a scintillation counter. If the label is a fluorescent label, the label is quantified by stimulating the label with a light of one color (which is known as the "excitation wavelength") and detecting another color (which is known as the "emission wavelength") that is emitted by the label in response to the stimulation. If the label is a chemiluminescent label, the label is quantified detecting the light emitted either visually or by using luminometers, x-ray film, high speed photographic film, a CCD camera, *etc.* If the amount of the label in the complex has been quantified, the concentration of target analyte in the test sample can be determined, *e.g*., by use of a standard curve that has been generated using serial dilutions of HIV-1 and/or HIV-2 core protein or fragments thereof known concentration. Other than using serial dilutions of target analyte(s), the standard curve can be generated gravimetrically, by mass spectroscopy and by other techniques known in the art.

The immunoassays described herein are intended to be illustrative and not limiting. Using the teaching provided herein numerous other immunoassays will be available to one of skill in the art. Such assays procedures, including those described above and below, are well-known in the art (see Immunological Methods, Vols. I and II, 1979 and 1981, Eds., Lefkovits and Pernis, Academic Press, New York; Antibodies, 1982, eds., Kennett et al., Plenum Press, New York; and Handbook of Experimental Immunology, 1978, ed., Weir, Blackwell Scientific Publications, St. Louis, Mo.).

It should be noted that the antibodies of the present invention preferably may be used either alone as a single capture antibody, or alone as a single probe and/or conjugated antibody. However, they may also be used in pairs or in trios in the assays described herein. Further, combinations of the antibodies of the present invention (and fragments thereof) may be used with other antibodies that have specificities for epitopes of HIV-1 and/or HIV-2, other than the epitope specificities of the antibodies of the present invention. Thus, the present antibodies may act as components in a mixture or "cocktail" of HIV-1 and/or HIV-2 antibodies. Thus, for example, this cocktail can include an antibody of the present invention which detects p24 of HIV-1 and p26 of HIV-2 and a antibody that detects a HIV envelope antigenic determinant in the transmembrane protein or extracellular glycoprotein. In this manner, one may be able to detect several antigenic determinants from different proteins of one or more viruses (*e.g*., HIV-1 and HIV-2) simultaneously.

Also, it should be noted that the antibodies of the present invention may be utilized in a combination assay which detects: 1) antigens, such as those described above (*e.g.,* p24 and p26) and 2) antibodies to HIV (by use of, for example, envelope antigens (*e.g.,* HIV-1 group M and O gp41 and HIV-2 gp36). Any such combination assay, which utilizes the antibodies of the present invention, is considered to be within the scope of the invention.

Examples of biological fluids which may be tested by the above immunoassays include, but are not limited to plasma, serum, cerebrospinal fluid, saliva, tears, nasal washes or aqueous extracts of tissues and cells. The test samples may also comprise inactivated whole virus or partially purified or recombinant p24 or p26 antigen.

It should also be noted that, in certain embodiments, the above-referenced antibodies may be used, when appropriately labeled, as competitive probes against HIV-1 and -2 core antibodies in serum samples for binding to recombinantly-derived HIV-1 p24 and HIV-2 p26.

Additionally, the antibodies of the present invention or fragments thereof may be used in detection systems using fixed cells or fixed tissues, with appropriate labeling of each antibody. In particular, the tissue sample is contacted with a conjugate comprising a signal-generating compound attached to one of the antibodies of the present invention in order to form a mixture. The mixture is then incubated for a time and under conditions sufficient for antigen/antibody complexes to form. The presence of antigen present in the sample is determined by detecting the signal generated . The antibodies may also be utilized for purifying HIV-1 p24 antigen and HIV-2 p26 antigen by, for example, affinity chromatography.

Furthermore, in certain aspects the antibodies of the invention may be bound to matrices and used for the affinity purification of specific HIV-1 and/or HIV-2 antigens from, for example, cell cultures, or biological tissues such as blood and liver. The antibodies, for example, may be attached to or immobilized on a substrate or support. The solution containing the HIV antigenic determinants is then contacted with the immobilized antibody for a time and under conditions suitable for the formation of immune complexes between the antibody and polypeptides containing the p24 and p26 determinants. Unbound material is separated from the bound immune complexes. The complexes or antigenic fragments are then separated from the support.

The anti-HIV antibodies described herein can also serve as research tools for epitope mapping of HIV proteins p24 and p26. Further, it should be noted that not only do the anti-HIV antibodies described herein bind to proteins and protein precursors of HIV clinical isolates which contain the targeted region or regions of antigenic determinants, in addition, the antibodies bind to recombinant proteins and synthetic analogues of the proteins which contain the antigenic determinant(s). Thus, for example, the antibodies described herein can be used in binding experiments involving recombinant proteins and synthetic analogues of p24 of HIV-1 and p26 of HIV-2.

Additionally, the antibodies described herein which are unlabeled may be used in agglutination assays or can be used in combination with labeled antibodies that are reactive with the antibody, such as antibodies specific for immunoglobulin.

### V. Pharmaceutical Compositions and Pharmaceutical Administration.

The antibodies described herein can be incorporated into pharmaceutical compositions suitable for administration to a subject. Typically, the pharmaceutical composition comprises a therapeutically or pharmaceutically effective amount of an anti-HIV antibody described herein along with a pharmaceutically acceptable carrier or excipient.

The antibodies can be administered in the "native" form or, if desired, in the form of salts, esters, amides, prodrugs, derivatives, and the like, provided the salt, ester, amide, prodrug or derivative is suitable pharmacologically, *i.e.,* effective in the present method(s). Salts, esters, amides, prodrugs and other derivatives of the active agents can be prepared using standard procedures known to those skilled in the art of synthetic organic chemistry and described, for example, by March (1992) Advanced Organic Chemistry; Reactions, Mechanisms and Structure, 4th Ed. N.Y. Wiley-Interscience.

Methods of formulating such derivatives are known to those of skill in the art. For example, the disulfide salts of a number of delivery agents are described in PCT Publication WO 2000/059863. Similarly, acid salts of therapeutic peptides, peptoids, or other mimetics, and can be prepared from the free base using conventional methodology that typically involves reaction with a suitable acid. Generally, the base form of the drug is dissolved in a polar organic solvent such as methanol or ethanol and the acid is added thereto. The resulting salt either precipitates or can be brought out of solution by addition of a less polar solvent. Suitable acids for preparing acid addition salts include, but are not limited to both organic acids, *e.g*., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids, *e.g.,* hydrochloric acid, hydrobromic acid, sulfuric acid, nitric acid, phosphoric acid, and the like. An acid addition salt can be reconverted to the free base by treatment with a suitable base. Certain particularly preferred acid addition salts of the active agents herein include halide salts, such as may be prepared using hydrochloric or hydrobromic acids. Conversely, preparation of basic salts of the active agents of this invention are prepared in a similar manner using a pharmaceutically acceptable base such as sodium hydroxide, potassium hydroxide, ammonium hydroxide, calcium hydroxide, trimethylamine, or the like. In certain embodiments basic salts include alkali metal salts, *e.g.,* the sodium salt, and copper salts.

For the preparation of salt forms of basic drugs, the pKa of the counterion is preferably at least about 2 pH lower than the pKa of the drug. Similarly, for the preparation of salt forms of acidic drugs, the pKa of the counterion is preferably at least about 2 pH higher than the pKa of the drug. This permits the counterion to bring the solution's pH to a level lower than the pHₘₐₓ to reach the salt plateau, at which the solubility of salt prevails over the solubility of free acid or base. The generalized rule of difference in pKa units of the ionizable group in the active pharmaceutical ingredient (API) and in the acid or base is meant to make the proton transfer energetically favorable. When the pKa of the API and counterion are not significantly different, a solid complex may form but may rapidly disproportionate (*i.e.,* break down into the individual entities of drug and counterion) in an aqueous environment.

In typical aspects the counterion is a pharmaceutically acceptable counterion. Suitable anionic salt forms include, but are not limited to acetate, benzoate, benzylate, bitartrate, bromide, carbonate, chloride, citrate, edetate, edisylate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate (embonate), phosphate and diphosphate, salicylate and disalicylate, stearate, succinate, sulfate, tartrate, tosylate, triethiodide, valerate, and the like, while suitable cationic salt forms include, but are not limited to aluminum, benzathine, calcium, ethylene diamine, lysine, magnesium, meglumine, potassium, procaine, sodium, tromethamine, zinc, and the like.

In various aspects preparation of esters typically involves functionalization of hydroxyl and/or carboxyl groups that are present within the molecular structure of the active agent. In certain embodiments, the esters are typically acyl-substituted derivatives of free alcohol groups, *i.e.,* moieties that are derived from carboxylic acids of the formula RCOOH where R is alky, and preferably is lower alkyl. Esters can be reconverted to the free acids, if desired, by using conventional hydrogenolysis or hydrolysis procedures.

Amides can also be prepared using techniques known to those skilled in the art or described in the pertinent literature. For example, amides may be prepared from esters, using suitable amine reactants, or they may be prepared from an anhydride or an acid chloride by reaction with ammonia or a lower alkyl amine.

As indicated above, in certain aspects the pharmaceutical composition comprises a therapeutically or pharmaceutically effective amount of an anti-HIV antibody described herein along with a pharmaceutically acceptable carrier or excipient. As used herein, "pharmaceutically acceptable carrier" or "pharmaceutically acceptable excipient" includes any and all solvents, dispersion media, coating, antibacterial and antifungal agents, isotonic and absorption delaying agents, and the like that are physiologically compatible.

Pharmaceutically acceptable carriers can contain one or more physiologically acceptable compound(s) that act, for example, to stabilize the composition or to increase or decrease the absorption of the active agent(s). Physiologically acceptable compounds can include, for example, carbohydrates, such as glucose, sucrose, or dextrans, antioxidants, such as ascorbic acid or glutathione, chelating agents, low molecular weight proteins, protection and uptake enhancers such as lipids, compositions that reduce the clearance or hydrolysis of the active agents, or excipients or other stabilizers and/or buffers.

Other physiologically acceptable compounds, particularly of use in the preparation of tablets, capsules, gel caps, and the like include, but are not limited to binders, diluent/fillers, disentegrants, lubricants, suspending agents, and the like.

In certain aspects to manufacture an oral dosage form (*e.g.,* a tablet), an excipient (*e.g.,* lactose, sucrose, starch, mannitol, *etc.*), an optional disintegrator (*e.g.* calcium carbonate, carboxymethylcellulose calcium, sodium starch glycollate, crospovidone *etc.*), a binder (*e.g.* alpha-starch, gum arabic, microcrystalline cellulose, carboxymethylcellulose, polyvinylpyrrolidone, hydroxypropylcellulose, cyclodextrin, *etc.*), and an optional lubricant (*e.g.,* talc, magnesium stearate, polyethylene glycol 6000, *etc.*), for instance, are added to the active component or components (*e.g*., active peptide) and the resulting composition is compressed. Where necessary the compressed product is coated, *e.g*., known methods for masking the taste or for enteric dissolution or sustained release. Suitable coating materials include, but are not limited to ethyl-cellulose, hydroxymethylcellulose, polyoxyethylene glycol, cellulose acetate phthalate, hydroxypropylmethylcellulose phthalate, and Eudragit (Rohm & Haas, Germany; methacrylic-acrylic copolymer).

Other physiologically acceptable compounds include wetting agents, emulsifying agents, dispersing agents or preservatives that are particularly useful for preventing the growth or action of microorganisms. Various preservatives are well known and include, for example, phenol and ascorbic acid. One skilled in the art would appreciate that the choice of pharmaceutically acceptable carrier(s), including a physiologically acceptable compound depends, for example, on the route of administration of the active agent(s) and on the particular physio-chemical characteristics of the active agent(s).

In certain aspects the excipients are sterile and generally free of undesirable matter. These compositions can be sterilized by conventional, well-known sterilization techniques. For various oral dosage form excipients such as tablets and capsules sterility is not required. The USP/NF standard is usually sufficient.

The pharmaceutical compositions may be in a variety of forms. They include, for example, liquid, semi-solid and solid dosage forms, such as liquid solutions (*e.g.* injectable and infusible solutions), dispersions or suspensions, tablets, pills, powders, liposomes and suppositories. The preferred form depends on the intended mode of administration and therapeutic application. Typical preferred compositions are in the form of injectable or infusible solutions, such as compositions similar to those used for passive immunization of humans with other antibodies. A typical mode of administration is parenteral (*e.g*., intravenous, subcutaneous, intraperitoneal, intramuscular). In certain embodiments, the antibody is administered by intravenous infusion or injection. In another embodiment, the antibody or antibody fragment is administered by intramuscular or subcutaneous injection.

The antibodies of the present invention can be administered by a variety of methods known in the art, although for many therapeutic applications, the preferred route/mode of administration is intravenous injection or infusion. As will be appreciated by those skilled in the art, the route and/or mode of administration will vary depending upon the desired results. In certain aspects the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art (*see, e.g.,* Robinson ed. (1978). Sustained and Controlled Release Drug Delivery Systems, Marcel Dekker, Inc., New York).

In certain aspects, the antibody may be orally administered, for example, with an inert diluent or an assimilable edible carrier. The compound (and other ingredients if desired) may also be enclosed in a hard or soft shell gelatin capsule, compressed into tablets, buccal tablets, troches, capsules, elixirs, suspensions, syrups, wafers, and the like. To administer an antibody or antibody fragment of the invention by other than parenteral administration, it may be necessary to coat the compound with, or co-administer the compound with, a material to prevent its inactivation.

Supplementary active compounds also can be incorporated into the compositions. In certain embodiments, the antibody or antibody portion is co-formulated with and/or co-administered with one or more additional therapeutic agents. Such combination therapies may advantageously utilize lower dosages of the administered therapeutic agents, thus avoiding possible toxicities or complications associated with monotherapies or alternatively, act synergistically or additively to enhance the therapeutic effect.

Dosage regimens may be adjusted to provide the optimum desired response (*e.g.,* a therapeutic or prophylactic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be tested; each unit containing a predetermined quantity of active compound calculated to produce the desired therapeutic effect in association with the required pharmaceutical carrier. The specification for the dosage unit forms of the present invention are dictated by and directly dependent on (a) the unique characteristics of the active compound and the particular therapeutic or prophylactic effect to be achieved and (b) the limitations inherent in the art of compounding such an active compound for the treatment of sensitivity in individuals.

An illustrative, non-limiting range for a therapeutically or prophylactically effective amount of an antibody or antibody portion of the invention is 0.1-20 mg/kg, more preferably 0.5-10 mg/kg. It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are illustrative only and are not intended to limit the scope or practice of the claimed composition.

### VI. Kits.

In another embodiment this kits are provided for the practice of any of the methods described herein. The kits typically comprise a container containing one or more of the antibodies described herein. In certain embodiments the antibodies are labeled with a detectable label. In certain embodiments the antibodies are provided in a pharmaceutical formulation (*e.g.* a unit dosage formulation such as a suppository, tablet, caplet, patch, *etc.*) and/or may be optionally combined with one or more pharmaceutically acceptable excipients.

In certain embodiments the kits further include one or more additional reagents to practice the methods described herein. Such reagents include, but are not limited to antibodies conjugated to detectable labels, substrates for signal generating compounds, buffers, substrates having antibodies attached thereto, serial dilutions of HIV-1 and/or HIV-2 proteins, and the like.

In addition, the kits optionally include labeling and/or instructional materials providing directions (*i.e.,* protocols) for the practice of the methods or use of the "therapeutics" or "prophylactics" or detection reagents of this invention. Certain instructional materials describe the use of one or more active agent(s) of this invention to therapeutically or prophylactically to inhibit or prevent infection. The instructional materials may also, optionally, teach preferred dosages/therapeutic regiment, counter indications and the like. Certain instructional materials provide protocols for practicing one or more of the assays described herein.

While the instructional materials typically comprise written or printed materials they are not limited to such. Any medium capable of storing such instructions and communicating them to an end user is contemplated by this invention. Such media include, but are not limited to electronic storage media (*e.g*., magnetic discs, tapes, cartridges, chips), optical media (*e.g.,* CD ROM), and the like. Such media may include addresses to internet sites that provide such instructional materials.

### EXAMPLES

The following examples are offered to illustrate, but not to limit the claimed invention.

### Example 1

### Determination of antibody binding affinity to antigens

Azlactone beads (obtained from Pierce, Rockford, IL) were coated with HIV antigens by mixing 50 mg of beads, 80 µg of HIV antigens (HIV-1 Group M, HIV-1 Group O, or HIV-2 rp26), 100 µL of 0.5 M carbonate buffer pH 10, and 900 µL of distilled water and rotating 1 hour at room temperature. The beads were then spun down by centrifuging 30s at 14,000 xg and the supernatant was aspirated off. The beads were resuspended in 900 µL of 1 M Trizma HCl buffer and 100 µL of 10% BSA and rotating 2 hours at room temperature to block free antigen linkage spots. The beads were washed twice with PBS pH 7.4 and finally resuspended in 30 mL PBS pH 7.4.

Sample solutions containing anti-HIV mAbs (fixed concentration) and series of HIV antigens with various concentrations were prepared as following. Anti-HIV mAbs were diluted to 1 pM in PBS pH 7.4 with 1% BSA. HIV antigens were diluted in 20 mL of 1 pM anti-HIV mAbs to reach a final concentration of 100 pM. Series of 2-fold dilutions of anti-HIV antigens were prepared by mixing equal volumes of antigen in 1 pM anti-HIV mAbs solution and 1 pM anti-HIV mAbs solution. The resultant series of anti-HIV mAbs and HIV antigens mixtures included antigen concentrations of 100, 50, 25, 12.5, 6.25, 3.13, 1.56, 0.79, 0.39, 0.20, 0.10, and 0.05 pM. The antigen-mAb sample solutions were incubated 8 hours at room temperature to let the binding reach equilibrium prior to testing on an instrument called KinExA (Sapidyne Instruments Inc., Boise, ID) for relative amount of free mAbs in solution phase.

The HIV antigen-coated azlactone beads were loaded in the KinExA bead reservoir. The instrument automatically drew 467 µL beads slurry from the bead reservoir and packed the beads in its flow cell. The sample lines of the instrument were placed in antigen-mAb sample solutions. The instrument then ran 4 mL of sample solution over the packed beads. The HIV antigen-coated beads captured a small portion of free mAb in sample solution without disturbing the binding equilibrium. After washing beads with PBS pH 7.4, the instrument ran 1.5 mL of 1 µg/ml goat anti-mouse Cy5 or goat anti-human Cy5 conjugate over the beads to bind to the mAbs captured on the beads. The differences in fluorescence before mAb capture and after conjugate binding were reported as signals.

The signal versus antigen concentration data were mathematically fitted using the least-square approach by the software provided with the instrument. The dissociation constant (K_{D}) was calculated and reported (*see* Table 4.

**Table 4. K_{D}, kₒₙ and k_{off} calculated for HIV antibodies.**

| | Antigen | | | | | | |
|---|---|---|---|---|---|---|---|
| | HIV-1 Group M | | | HIV-1 Group O | | | HIV2 rp26 |
| Ab | K_{D} (pM) | kₒₙ (M⁻¹s⁻¹) | k_{off} (s⁻¹) | K_{D} (pM) | kₒₙ (M⁻¹s⁻¹) | k_{off} (s⁻¹) | K_{D} (pM) |
| WT | 8.8 | 0.81x10⁷ | 7.1x10⁻⁵ | 10.0 | 1.4x10⁷ | 1.4x10⁻⁴ | 10.8 |
| AM1 | 0.96 | 1.1x10⁷ | 1.1x10⁻⁵ | 0.27 | 1.7x10⁷ | 4.6x10⁻⁶ | 0.84 |
| AM2 | 0.62 | 1.1x10⁷ | 6.8x10⁻⁶ | 0.58 | 1.7x10⁷ | 9.9x10⁻⁶ | 0.79 |

| | | | | | | | |
|---|---|---|---|---|---|---|---|
| * Off-rates (koff) are calculated from K_{D} and kₒₙ. ND: not determined. N/A: not applicable | | | | | | | |

### SEQUENCE LISTING

(SEQ ID NO:44) **115B-151-423 AM1 mG1k**
   **Nucleotide sequence of heavy chain** 
CAGGTTCAGCTGCAGCAGTCTGGAGCTGAACTGATGAAGCCTGGGGCCTCAGTGAAGATA
TCCTGCAAGGCTTCTGGATACACATTCACTAGCTACTGGATAGAGTGGATAAAACAGAGG
CCTGGACATGGCCTTGAGTGGATTGGAGAGATTTTGCCTGGAGCTGGTAGTCTTAACAAC
AATGAGAAGTTCAGGGACAAGGCCACATTCACTGCTGATACTTCCTCCAACACAGCCTAC
ATGCAACTCAGCAGCCTGACATCTGAGGACTCTGCCGTCTATTACTGTGCAAGAGGTTAC
CGTTACGACGGCATCATGTTCTATTGGGGCCAAGGGACTCTGGTCACTGTCTCTGCAGCC
AAAACGACACCCCCATCTGTCTATCCACTGGCCCCTGGATCTGCTGCCCAAACTAACTCC
ATGGTGACCCTGGGATGCCTGGTCAAGGGCTATTTCCCTGAGCCAGTGACAGTGACCTGG
AACTCTGGATCCCTGTCCAGCGGTGTGCACACCTTCCCAGCTGTCCTGCAGTCTGACCTC
TACACTCTGAGCAGCTCAGTGACTGTCCCCTCCAGCACCTGGCCCAGCGAGACCGTCACC
TGCAACGTTGCCCACCCGGCCAGCAGCACCAAGGTGGACAAGAAAATTGTGCCCAGGGAT
TGTGGTTGTAAGCCTTGCATATGTACAGTCCCAGAAGTATCATCTGTCTTCATCTTCCCC
CCAAAGCCCAAGGATGTGCTCACCATTACTCTGACTCCTAAGGTCACGTGTGTTGTGGTA
GACATCAGCAAGGATGATCCCGAGGTCCAGTTCAGCTGGTTTGTAGATGATGTGGAGGTG
CACACAGCTCAGACGCAACCCCGGGAGGAGCAGTTCAACAGCACTTTCCGCTCAGTCAGT
GAACTTCCCATCATGCACCAGGACTGGCTCAATGGCAAGGAGTTCAAATGCAGGGTCAAC
AGTGCAGCTTTCCCTGCCCCCATCGAGAAAACCATCTCCAAAACCAAAGGCAGACCGAAG
GCTCCACAGGTGTACACCATTCCACCTCCCAAGGAGCAGATGGCCAAGGATAAAGTCAGT
CTGACCTGCATGATAACAGACTTCTTCCCTGAAGACATTACTGTGGAGTGGCAGTGGAAT
GGGCAGCCAGCGGAGAACTACAAGAACACTCAGCCCATCATGGACACAGATGGCTCTTAC
TTCGTCTACAGCAAGCTCAATGTGCAGAAGAGCAACTGGGAGGCAGGAAATACTTTCACC
TGCTCTGTGTTACATGAGGGCCTGCACAACCACCATACTGAGAAGAGCCTCTCCCACTCT
CCTGGTAAATAA
**(SEQ ID NO:45) Nucleotide sequence of light chain** 
GACATCCAGATGACTCAGTCTCCAGCCTCCCTATCTGCATCTGTGGGAGAAACTGTCACC
ATCACATGTCGAACAAGTGAGAATATTTACAGTTATTTAGCATGGTATCAGCAGAAACCG
GGAAAATCTCCTCACCTCCTGGTCTATAATACAAAGACCTTAGCAGAAGGTGTGCCATCT
AGGTTCAGTGGCAGTGGATCAGGAACACAATTTTCTCTGAAGATCAACAGCCTGCAGCCT
GAAGATTTTGGGAGTTATTACTGTCAACATCATTATGATGAGGTGCTGACGTTCGGTTCT
GGGACCAAGCTGGAACTGAAACGGGCTGATGCTGCACCAACTGTATCCATCTTCCCACCA
TCCAGTGAGCAGTTAACATCTGGAGGTGCCTCAGTCGTGTGCTTCTTGAACAACTTCTAC
CCCAAAGACATCAATGTCAAGTGGAAGATTGATGGCAGTGAACGACAAAATGGCGTCCTG
AACAGTTGGACTGATCAGGACAGCAAAGACAGCACCTACAGCATGAGCAGCACCCTCACG
TTGACCAAGGACGAGTATGAACGACATAACAGCTATACCTGTGAGGCCACTCACAAGACA
TCAACTTCACCCATTGTCAAGAGCTTCAACAGGAATGAGTGTTAA
**(SEQ ID NO:46) 115B-151-423 AM1 mG1k**
   **Amino acid sequence of heavy chain** 
QVQLQQSGAELMKPGASVKISCKASGYTFTSYWIEWIKQRPGHGLEWIGEILPGAGSLNN
NEKFRDKATFTADTSSNTAYMQLSSLTSEDSAVYYCARGYRYDGIMFYWGQGTLVTVSAA
KTTPPSVYPLAPGSAAQTNSMVTLGCLVKGYFPEPVTVTWNSGSLSSGVHTFPAVLQSDL
YTLSSSVTVPSSTWPSETVTCNVAHPASSTKVDKKIVPRDCGCKPCICTVPEVSSVFIFP
PKPKDVLTITLTPKVTCVVVDISKDDPEVQFSWFVDDVEVHTAQTQPREEQFNSTFRSVS
ELPIMHQDWLNGKEFKCRVNSAAFPAPIEKTISKTKGRPKAPQVYTIPPPKEQMAKDKVS
LTCMITDFFPEDITVEWQWNGQPAENYKNTQPIMDTDGSYFVYSKLNVQKSNWEAGNTFT
CSVLHEGLHNHHTEKSLSHSPGK
**(SEQ ID NO:47) Amino acid sequence of light chain** 
DIQMTQSPASLSASVGETVTITCRTSENIYSYLAWYQQKPGKSPHLLVYNTKTLAEGVPS
RFSGSGSGTQFSLKINSLQPEDFGSYYCQHHYDEVLTFGSGTKLELKRADAAPTVSIFPP
SSEQLTSGGASVVCFLNNFYPKDINVKWKIDGSERQNGVLNSWTDQDSKDSTYSMSSTLT
LTKDEYERHNSYTCEATHKTSTSPIVKSFNRNEC
(SEQ ID NO:48) **115B-151-423 AM2 mGlk**
   **Nucleotide sequence of heavy chain** 
CAGGTTCAGCTGCAGCAGTCTGGAGCTGAACTGATGAAGCCTGGGGCCTCAGTGAAGATA
TCCTGCAAGGCTTCTGGATACACATTCACTAGCTACTGGATAGAGTGGATAAAACAGAGG
CCTGGACATGGCCTTGAGTGGATTGGAGAGATTTTGCCTGGAACTGGTAGTCTTAACAAC
AATGAGAAGTTCAGGGACAAGGCCACATTCACTGCTGATACTTCCTCCAACACAGCCTAC
ATGCAACTCAGCAGCCTGACATCTGAGGACTCTGCCGTCTATTACTGTGCAAGAGGTTAC
CGTTACGACGGCATCATGTTCTATTGGGGCCAAGGGACTCTGGTCACTGTCTCTGCAGCC
AAAACGACACCCCCATCTGTCTATCCACTGGCCCCTGGATCTGCTGCCCAAACTAACTCC
ATGGTGACCCTGGGATGCCTGGTCAAGGGCTATTTCCCTGAGCCAGTGACAGTGACCTGG
AACTCTGGATCCCTGTCCAGCGGTGTGCACACCTTCCCAGCTGTCCTGCAGTCTGACCTC
TACACTCTGAGCAGCTCAGTGACTGTCCCCTCCAGCACCTGGCCCAGCGAGACCGTCACC
TGCAACGTTGCCCACCCGGCCAGCAGCACCAAGGTGGACAAGAAAATTGTGCCCAGGGAT
TGTGGTTGTAAGCCTTGCATATGTACAGTCCCAGAAGTATCATCTGTCTTCATCTTCCCC
CCAAAGCCCAAGGATGTGCTCACCATTACTCTGACTCCTAAGGTCACGTGTGTTGTGGTA
GACATCAGCAAGGATGATCCCGAGGTCCAGTTCAGCTGGTTTGTAGATGATGTGGAGGTG
CACACAGCTCAGACGCAACCCCGGGAGGAGCAGTTCAACAGCACTTTCCGCTCAGTCAGT
GAACTTCCCATCATGCACCAGGACTGGCTCAATGGCAAGGAGTTCAAATGCAGGGTCAAC
AGTGCAGCTTTCCCTGCCCCCATCGAGAAAACCATCTCCAAAACCAAAGGCAGACCGAAG
GCTCCACAGGTGTACACCATTCCACCTCCCAAGGAGCAGATGGCCAAGGATAAAGTCAGT
CTGACCTGCATGATAACAGACTTCTTCCCTGAAGACATTACTGTGGAGTGGCAGTGGAAT
GGGCAGCCAGCGGAGAACTACAAGAACACTCAGCCCATCATGGACACAGATGGCTCTTAC
TTCGTCTACAGCAAGCTCAATGTGCAGAAGAGCAACTGGGAGGCAGGAAATACTTTCACC
TGCTCTGTGTTACATGAGGGCCTGCACAACCACCATACTGAGAAGAGCCTCTCCCACTCT
CCTGGTAAATAA
**(SEQ ID NO:49) Nucleotide sequence of light chain** 
GACATCCAGATGACTCAGTCTCCAGCCTCCCTATCTGCATCTGTGGGAGAAACTGTCACC
ATCACATGTCGAACAAGTGAGAATATTTACAGTTATTTAGCATGGTATCAGCAGAAACCG
GGAAAATCTCCTCACCTCCTGGTCTATAATACAAAGACCTTAGCAGAAGGTGTGCCATCT
AGGTTCAGTGGCAGTGGATCAGGAACACAATTTTCTCTGAAGATCAACAGCCTGCAGCCT
GAAGATTTTGGGAGTTATTACTGTCAACATCATTATGATGAGGTGCTGACGTTCGGTTCT
GGGACCAAGCTGGAACTGAAACGGGCTGATGCTGCACCAACTGTATCCATCTTCCCACCA
TCCAGTGAGCAGTTAACATCTGGAGGTGCCTCAGTCGTGTGCTTCTTGAACAACTTCTAC
CCCAAAGACATCAATGTCAAGTGGAAGATTGATGGCAGTGAACGACAAAATGGCGTCCTG
AACAGTTGGACTGATCAGGACAGCAAAGACAGCACCTACAGCATGAGCAGCACCCTCACG
TTGACCAAGGACGAGTATGAACGACATAACAGCTATACCTGTGAGGCCACTCACAAGACA
TCAACTTCACCCATTGTCAAGAGCTTCAACAGGAATGAGTGTTAA
(SEQ ID NO:50) **115B-151-423 AM2 mG1k**
   **Amino acid sequence of heavy chain** 
QVQLQQSGAELMKPGASVKISCKASGYTFTSYWIEWIKQRPGHGLEWIGEILPGTGSLNN
NEKFRDKATFTADTSSNTAYMQLSSLTSEDSAVYYCARGYRYDGIMFYWGQGTLVTVSAA
KTTPPSVYPLAPGSAAQTNSMVTLGCLVKGYFPEPVTVTWNSGSLSSGVHTFPAVLQSDL
YTLSSSVTVPSSTWPSETVTCNVAHPASSTKVDKKIVPRDCGCKPCICTVPEVSSVFIFP
PKPKDVLTITLTPKVTCVVVDISKDDPEVQFSWFVDDVEVHTAQTQPREEQFNSTFRSVS
ELPIMHQDWLNGKEFKCRVNSAAFPAPIEKTISKTKGRPKAPQVYTIPPPKEQMAKDKVS
LTCMITDFFPEDITVEWQWNGQPAENYKNTQPIMDTDGSYFVYSKLNVQKSNWEAGNTFT
CSVLHEGLHNHHTEKSLSHSPGK
**(SEQ ID NO:51) Amino acid sequence of light chain** 
DIQMTQSPASLSASVGETVTITCRTSENIYSYLAWYQQKPGKSPHLLVYNTKTLAEGVPS
RFSGSGSGTQFSLKINSLQPEDFGSYYCQHHYDEVLTFGSGTKLELKRADAAPTVSIFPP
SSEQLTSGGASVVCFLNNFYPKDINVKWKIDGSERQNGVLNSWTDQDSKDSTYSMSSTLT
LTKDEYERHNSYTCEATHKTSTSPIVKSFNRNEC
(SEQ ID NO:52) **115B-151-423 AM1 hG1k**
   **Nucleotide sequence of heavy chain** 
CAGGTTCAGCTGCAGCAGTCTGGAGCTGAACTGATGAAGCCTGGGGCCTCAGTGAAGATA
TCCTGCAAGGCTTCTGGATACACATTCACTAGCTACTGGATAGAGTGGATAAAACAGAGG
CCTGGACATGGCCTTGAGTGGATTGGAGAGATTTTGCCTGGAGCTGGTAGTCTTAACAAC
AATGAGAAGTTCAGGGACAAGGCCACATTCACTGCTGATACTTCCTCCAACACAGCCTAC
ATGCAACTCAGCAGCCTGACATCTGAGGACTCTGCCGTCTATTACTGTGCAAGAGGTTAC
CGTTACGACGGCATCATGTTCTATTGGGGCCAAGGGACTCTGGTCACTGTCTCTGCAGCG
TCGACCAAGGGCCCATCGGTCTTCCCCCTGGCACCCTCCTCCAAGAGCACCTCTGGGGGC
ACAGCGGCCCTGGGCTGCCTGGTCAAGGACTACTTCCCCGAACCGGTGACGGTGTCGTGG
AACTCAGGCGCCCTGACCAGCGGCGTGCACACCTTCCCGGCTGTCCTACAGTCCTCAGGA
CTCTACTCCCTCAGCAGCGTGGTGACCGTGCCCTCCAGCAGCTTGGGCACCCAGACCTAC
ATCTGCAACGTGAATCACAAGCCCAGCAACACCAAGGTGGACAAGAAAGTTGAGCCCAAA
TCTTGTGACAAAACTCACACATGCCCACCGTGCCCAGCACCTGAACTCCTGGGGGGACCG
TCAGTCTTCCTCTTCCCCCCAAAACCCAAGGACACCCTCATGATCTCCCGGACCCCTGAG
GTCACATGCGTGGTGGTGGACGTGAGCCACGAAGACCCTGAGGTCAAGTTCAACTGGTAC
GTGGACGGCGTGGAGGTGCATAATGCCAAGACAAAGCCGCGGGAGGAGCAGTACAACAGC
ACGTACCGTGTGGTCAGCGTCCTCACCGTCCTGCACCAGGACTGGCTGAATGGCAAGGAG
TACAAGTGCAAGGTCTCCAACAAAGCCCTCCCAGCCCCCATCGAGAAAACCATCTCCAAA
GCCAAAGGGCAGCCCCGAGAACCACAGGTGTACACCCTGCCCCCATCCCGCGAGGAGATG
ACCAAGAACCAGGTCAGCCTGACCTGCCTGGTCAAAGGCTTCTATCCCAGCGACATCGCC
GTGGAGTGGGAGAGCAATGGGCAGCCGGAGAACAACTACAAGACCACGCCTCCCGTGCTG
GACTCCGACGGCTCCTTCTTCCTCTACAGCAAGCTCACCGTGGACAAGAGCAGGTGGCAG
CAGGGGAACGTCTTCTCATGCTCCGTGATGCATGAGGCTCTGCACAACCACTACACGCAG
AAGAGCCTCTCCCTGTCTCCGGGTAAATGA
**(SEQ ID NO:53) Nucleotide sequence of light chain** 
GACATCCAGATGACTCAGTCTCCAGCCTCCCTATCTGCATCTGTGGGAGAAACTGTCACC
ATCACATGTCGAACAAGTGAGAATATTTACAGTTATTTAGCATGGTATCAGCAGAAACCG
GGAAAATCTCCTCACCTCCTGGTCTATAATACAAAGACCTTAGCAGAAGGTGTGCCATCT
AGGTTCAGTGGCAGTGGATCAGGAACACAATTTTCTCTGAAGATCAACAGCCTGCAGCCT
GAAGATTTTGGGAGTTATTACTGTCAACATCATTATGATGAGGTGCTGACGTTCGGTTCT
GGGACCAAGCTGGAACTGAAACGGCGTACGGTGGCTGCACCATCTGTCTTCATCTTCCCG
CCATCTGATGAGCAGTTGAAATCTGGAACTGCCTCTGTTGTGTGCCTGCTGAATAACTTC
TATCCCAGAGAGGCCAAAGTACAGTGGAAGGTGGATAACGCCCTCCAATCGGGTAACTCC
CAGGAGAGTGTCACAGAGCAGGACAGCAAGGACAGCACCTACAGCCTCAGCAGCACCCTG
ACGCTGAGCAAAGCAGACTACGAGAAACACAAAGTCTACGCCTGCGAAGTCACCCATCAG
GGCCTGAGCTCGCCCGTCACAAAGAGCTTCAACAGGGGAGAGTGTTGA
(SEQ ID NO:54) **115B-151-423 AM1 hG1k**
   **Amino acid sequence of heavy chain** 
QVQLQQSGAELMKPGASVKISCKASGYTFTSYWIEWIKQRPGHGLEWIGEILPGAGSLNN
NEKFRDKATFTADTSSNTAYMQLSSLTSEDSAVYYCARGYRYDGIMFYWGQGTLVTVSAA
STKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGP
SVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSREEM
TKNQVSLTCLVKGFYPSDIAVEWESNGQPENNYKTTPPVLDSDGSFFLYSKLTVDKSRWQ
QGNVFSCSVMHEALHNHYTQKSLSLSPGK
**(SEQ ID NO:55 Amino acid sequence of light chain** 
DIQMTQSPASLSASVGETVTITCRTSENIYSYLAWYQQKPGKSPHLLVYNTKTLAEGVPS
RFSGSGSGTQFSLKINSLQPEDFGSYYCQHHYDEVLTFGSGTKLELKRRTVAAPSVFIFP
PSDEQLKSGTASVVCLLNNFYPREAKVQWKVDNALQSGNSQESVTEQDSKDSTYSLSSTL
TLSKADYEKHKVYACEVTHQGLSSPVTKSFNRGEC
(SEQ ID NO:56) **115B-151-423 AM2 hG1k**
   **Nucleotide sequence of heavy chain** 
CAGGTTCAGCTGCAGCAGTCTGGAGCTGAACTGATGAAGCCTGGGGCCTCAGTGAAGATA
TCCTGCAAGGCTTCTGGATACACATTCACTAGCTACTGGATAGAGTGGATAAAACAGAGG
CCTGGACATGGCCTTGAGTGGATTGGAGAGATTTTGCCTGGAACTGGTAGTCTTAACAAC
AATGAGAAGTTCAGGGACAAGGCCACATTCACTGCTGATACTTCCTCCAACACAGCCTAC
ATGCAACTCAGCAGCCTGACATCTGAGGACTCTGCCGTCTATTACTGTGCAAGAGGTTAC
CGTTACGACGGCATCATGTTCTATTGGGGCCAAGGGACTCTGGTCACTGTCTCTGCAGCG
TCGACCAAGGGCCCATCGGTCTTCCCCCTGGCACCCTCCTCCAAGAGCACCTCTGGGGGC
ACAGCGGCCCTGGGCTGCCTGGTCAAGGACTACTTCCCCGAACCGGTGACGGTGTCGTGG
AACTCAGGCGCCCTGACCAGCGGCGTGCACACCTTCCCGGCTGTCCTACAGTCCTCAGGA
CTCTACTCCCTCAGCAGCGTGGTGACCGTGCCCTCCAGCAGCTTGGGCACCCAGACCTAC
ATCTGCAACGTGAATCACAAGCCCAGCAACACCAAGGTGGACAAGAAAGTTGAGCCCAAA
TCTTGTGACAAAACTCACACATGCCCACCGTGCCCAGCACCTGAACTCCTGGGGGGACCG
TCAGTCTTCCTCTTCCCCCCAAAACCCAAGGACACCCTCATGATCTCCCGGACCCCTGAG
GTCACATGCGTGGTGGTGGACGTGAGCCACGAAGACCCTGAGGTCAAGTTCAACTGGTAC
GTGGACGGCGTGGAGGTGCATAATGCCAAGACAAAGCCGCGGGAGGAGCAGTACAACAGC
ACGTACCGTGTGGTCAGCGTCCTCACCGTCCTGCACCAGGACTGGCTGAATGGCAAGGAG
TACAAGTGCAAGGTCTCCAACAAAGCCCTCCCAGCCCCCATCGAGAAAACCATCTCCAAA
GCCAAAGGGCAGCCCCGAGAACCACAGGTGTACACCCTGCCCCCATCCCGCGAGGAGATG
ACCAAGAACCAGGTCAGCCTGACCTGCCTGGTCAAAGGCTTCTATCCCAGCGACATCGCC
GTGGAGTGGGAGAGCAATGGGCAGCCGGAGAACAACTACAAGACCACGCCTCCCGTGCTG
GACTCCGACGGCTCCTTCTTCCTCTACAGCAAGCTCACCGTGGACAAGAGCAGGTGGCAG
CAGGGGAACGTCTTCTCATGCTCCGTGATGCATGAGGCTCTGCACAACCACTACACGCAG
AAGAGCCTCTCCCTGTCTCCGGGTAAATGA
**(SEQ ID NO:57) Nucleotide sequence of light chain** 
GACATCCAGATGACTCAGTCTCCAGCCTCCCTATCTGCATCTGTGGGAGAAACTGTCACC
ATCACATGTCGAACAAGTGAGAATATTTACAGTTATTTAGCATGGTATCAGCAGAAACCG
GGAAAATCTCCTCACCTCCTGGTCTATAATACAAAGACCTTAGCAGAAGGTGTGCCATCT
AGGTTCAGTGGCAGTGGATCAGGAACACAATTTTCTCTGAAGATCAACAGCCTGCAGCCT
GAAGATTTTGGGAGTTATTACTGTCAACATCATTATGATGAGGTGCTGACGTTCGGTTCT
GGGACCAAGCTGGAACTGAAACGGCGTACGGTGGCTGCACCATCTGTCTTCATCTTCCCG
CCATCTGATGAGCAGTTGAAATCTGGAACTGCCTCTGTTGTGTGCCTGCTGAATAACTTC
TATCCCAGAGAGGCCAAAGTACAGTGGAAGGTGGATAACGCCCTCCAATCGGGTAACTCC
CAGGAGAGTGTCACAGAGCAGGACAGCAAGGACAGCACCTACAGCCTCAGCAGCACCCTG
ACGCTGAGCAAAGCAGACTACGAGAAACACAAAGTCTACGCCTGCGAAGTCACCCATCAG
GGCCTGAGCTCGCCCGTCACAAAGAGCTTCAACAGGGGAGAGTGTTGA
(SEQ ID NO:58) **115B-151-423 AM2 hG1k**
   **Amino acid sequence of heavy chain** 
QVQLQQSGAELMKPGASVKISCKASGYTFTSYWIEWIKQRPGHGLEWIGEILPGTGSLNN
NEKFRDKATFTADTSSNTAYMQLSSLTSEDSAVYYCARGYRYDGIMFYWGQGTLVTVSAA
STKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGP
SVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSREEM
TKNQVSLTCLVKGFYPSDIAVEWESNGQPENNYKTTPPVLDSDGSFFLYSKLTVDKSRWQ
QGNVFSCSVMHEALHNHYTQKSLSLSPGK
**(SEQ ID NO:59) Amino acid sequence of light chain**

DIQMTQSPASLSASVGETVTITCRTSENIYSYLAWYQQKPGKSPHLLVYNTKTLAEGVPS
RFSGSGSGTQFSLKINSLQPEDFGSYYCQHHYDEVLTFGSGTKLELKRRTVAAPSVFIFP
PSDEQLKSGTASVVCLLNNFYPREAKVQWKVDNALQSGNSQESVTEQDSKDSTYSLSSTL
TLSKADYEKHKVYACEVTHQGLSSPVTKSFNRGEC

## Claims

1. An isolated antibody that binds to an HIV-1 and HIV-2 core protein, wherein said antibody comprises
(a) a heavy chain comprising the amino acid sequence of SEQ ID NO:54 or SEQ ID NO:58 and a light chain comprising the amino acid sequence of SEQ ID NO:55 or SEQ ID NO:59, or
(b) a heavy chain comprising the amino acid sequence of SEQ ID NO:46 or SEQ ID NO:50 and a light chain comprising the amino acid sequence of SEQ ID NO:47 or SEQ ID NO:51,
wherein said antibody immunospecifically binds to p24 with an equilibrium dissociation constant (K_{D}) of less than 7 pM.

2. The antibody according to claim 1, wherein said antibody is selected from the group consisting of a monoclonal antibody, a multispecific antibody an animal antibody, and a recombinant antibody.

3. The antibody according to claims 1[[,]] or 2, wherein said antibody is a mouse monoclonal antibody.

4. The antibody according to any one of claims 1, 2, or 3 wherein said antibody is attached to a solid substrate.

5. The antibody according to any one of claims 1, 2, or 3 for use in detecting the presence of one or more antigens selected from the group consisting of HIV-1 antigen and HIV-2 antigen, in a test sample, said method comprising:
contacting said test sample with said antibody which binds to Human Immunodeficiency Virus-1 protein p24 and Human Immunodeficiency Virus-2 protein p26 for a time and under conditions sufficient for the formation of an antibody/antigen complex; and
detecting said antibody/antigen complex, where the presence of said complex indicates the presence of at least HIV-1 or HIV2 antigen in said test sample.

6. The antibody for the use according to claim 5, wherein said antibody is labeled and said detecting comprises detecting said label.

7. The antibody for the use according to claim 5, further comprising:
contacting a conjugate to said antibody/antigen complex for a time and under conditions sufficient to allow said conjugate to bind to the bound antigen comprising said complex, wherein said conjugate comprises a second antibody attached to a signal generating compound capable of generating a detectable signal; and
detecting presence of antigen present in said test sample by detecting a signal generated by said signal generating compound, the presence or strength of said signal indicating the presence of at least one antigen selected from the group consisting of HIV-1 antigen and HIV-2 antigen in said test sample.

8. A kit for detecting the presence of one or more antigens selected from the group consisting of HIV-1 antigen and HIV-2 antigen, in a test sample said kit comprising:
a first antibody that binds to HIV-1 p24 antigen and HIV-2 p26 antigen; and/or
an indicator reagent comprising a second antibody that binds to HIV-1 p24 antigen and HIV-2 p26 antigen to which a signal generating compound is attached; wherein said first antibody and/or said second antibody is an antibody according to any one of claims 1, 2, or 3.

9. The isolated antibody of claim 1, which comprises a heavy chain comprising the amino acid sequence of SEQ ID NO:54 or SEQ ID NO:58 and a light chain comprising the amino acid sequence of SEQ ID NO:55 or SEQ ID NO:59.

10. The isolated antibody of claim 1, which comprises a heavy chain comprising the amino acid sequence of SEQ ID NO:46 or SEQ ID NO:50 and a light chain comprising the amino acid sequence of SEQ ID NO:47 or SEQ ID NO:51.

## Patentansprüche

1. Ein isolierter Antikörper, der an HIV-1- und HIV-2-Kern-Protein bindet, worin der genannte Antikörper Folgendes umfasst:
(a) eine schwere Kette umfassend die Aminosäuresequenz von SEQUENZIDENTIFIKATIONSNR.: 54 oder SEQUENZIDENTIFIKATIONSNR.: 58 und eine leichte Kette umfassend die Aminosäuresequenz von SEQUENZIDENTIFIKATIONSNR.: 55 oder SEQUENZIDENTIFIKATIONSNR.: 59, oder
(b) eine schwere Kette umfassend die Aminosäuresequenz von SEQUENZIDENTIFIKATIONSNR.: 46 oder SEQUENZIDENTIFIKATIONSNR.: 50 und eine leichte Kette umfassend die Aminosäuresequenz von SEQUENZIDENTIFIKATIONSNR.: 47 oder SEQUENZIDENTIFIKATIONSNR.: 51,
worin der genannte Antikörper immunspezifisch an p24 mit einer Gleichgewichtsdissoziationskonstante (K_{D}) von weniger als 7 pM bindet.

2. Der Antikörper gemäß Anspruch 1, worin der genannte Antikörper gewählt ist aus der Gruppe bestehend aus einem monoklonalen Antikörper, einem multispezifischen Antikörper, einem tierischen Antikörper, und einem rekombinanten Antikörper.

3. Der Antikörper gemäß Ansprüchen 1 oder 2, worin der genannte Antikörper ein Maus-monoklonaler Antikörper ist.

4. Der Antikörper gemäß irgendeinem der Ansprüche 1, 2 oder 3, worin der Antikörper an ein festes Substrat angeheftet ist.

5. Der Antikörper gemäß irgendeinem der Ansprüche 1, 2 oder 3, für die Verwendung in der Detektion des Vorhandenseins von einem oder mehreren Antigenen gewählt aus der Gruppe bestehend aus HIV-1-Antigen und HIV-2-Antigen in einer Testprobe, wobei das Verfahren Folgendes umfasst:
In-Kontakt-bringen der genannten Testprobe mit dem genannten Antikörper, welcher an menschliches Immundefizienz-Virus-1 Protein p24 und menschliches Immundefizienz-Virus-2 Protein p26 bindet, für einen Zeitraum und unter Bedingungen, die ausreichend sind für die Bildung eines Antikörper/Antigen-Komplexes; und
Detektieren des Antikörper/Antigen-Komplexes, worin das Vorhandensein des genannten Komplexes das Vorhandensein von mindestens HIV-1- oder HIV-2-Antigen in der genannten Testprobe anzeigt.

6. Der Antikörper für die Verwendung gemäß Anspruch 5, worin der genannte Antikörper markiert ist und die genannte Detektion das Detektieren des genannten Markers umfasst.

7. Der Antikörper für die Verwendung gemäß Anspruch 5, weiter umfassend:
In-Kontakt-bringen eines Konjugats mit dem genannten Antikörper/Antigen-Komplex für einen Zeitraum und unter Bedingungen, die ausreichend sind, damit das genannte Konjugat an das gebundene Antigen, umfassend den genannten Komplex, bindet, worin das genannte Konjugat einen zweiten Antikörper umfasst, angeheftet an eine signalerzeugende Verbindung, die in der Lage ist, ein detektierbares Signal zu erzeugen; und
Detektieren der Anwesenheit von Antigen, welches in der genannten Testprobe vorhanden ist, durch Detektieren eines Signals, das durch die genannte signalerzeugende Verbindung erzeugt wurde, wobei das Vorhandensein oder die Stärke des genannten Signals das Vorhandensein von mindestens einem Antigen gewählt aus der Gruppe bestehend aus HIV-1-Antigen und HIV-2-Antigen in der genannten Testprobe anzeigt.

8. Ein Kit zur Detektion des Vorhandenseins von einem oder mehreren Antigenen gewählt aus der Gruppe bestehend aus HIV-1-Antigen und HIV-2-Antigen, in einer Testprobe, wobei der Kit folgendes umfasst:
einen ersten Antikörper, der an HIV-1 p24 Antigen und HIV-2 p26 Antigen bindet; und/oder
ein Indikatorreagenz umfassend einen zweiten Antikörper, der an HIV-1 p24 Antigen und HIV-2 p26 Antigen bindet, an welchen eine signalerzeugende Verbindung angeheftet ist; worin der genannte erste Antikörper und/oder der genannte zweite Antikörper ein Antikörper gemäß irgendeinem der Ansprüche 1, 2 oder 3 ist.

9. Der isolierte Antikörper gemäß Anspruch 1, welcher eine schwere Kette umfassend die Aminosäuresequenz von SEQUENZIDENTIFIKATIONSNR.: 54 oder SEQUENZIDENTIFIKATIONSNR.: 58 und eine leichte Kette umfassend die Aminosäuresequenz von SEQUENZIDENTIFIKATIONSNR.: 55 oder SEQUENZIDENTIFIKATIONSNR.: 59 umfasst.

10. Der isolierte Antikörper gemäß Anspruch 1, welcher eine schwere Kette umfassend die Aminosäuresequenz von SEQUENZIDENTIFIKATIONSNR.: 46 oder SEQUENZIDENTIFIKATIONSNR.: 50 und eine leichte Kette umfassend die Aminosäuresequenz von SEQUENZIDENTIFIKATIONSNR.: 47 oder SEQUENZIDENTIFIKATIONSNR,; 51 umfasst.

## Revendications

1. Anticorps isolé qui se lie à une protéine nucléocapsidique du VIH-1 et du VIH-2, où ledit anticorps comprend
(a) une chaîne lourde comprenant la séquence d'acides aminés de SEQ ID NO : 54 ou SEQ ID NO : 58 et une chaîne légère comprenant la séquence d'acides aminés de SEQ ID NO : 55 ou SEQ ID NO : 59, ou
(b) une chaîne lourde comprenant la séquence d'acides aminés de SEQ ID NO : 46 ou SEQ ID NO : 50 et une chaîne légère comprenant la séquence d'acides aminés de SEQ ID NO : 47 ou SEQ ID NO : 51,
où ledit anticorps se lie de façon immunospécifique à la p24 avec une constante de dissociation à l'équilibre (K_{D}) inférieure à 7 pM.

2. Anticorps selon la revendication 1, où ledit anticorps est choisi dans le groupe constitué d'un anticorps monoclonal, un anticorps multispécifique, un anticorps animal, et un anticorps recombinant.

3. Anticorps selon les revendications 1 ou 2, où ledit anticorps est un anticorps monoclonal de souris.

4. Anticorps selon l'une quelconque des revendications 1, 2, ou 3, où ledit anticorps est fixé à un substrat solide.

5. Anticorps selon l'une quelconque des revendications 1, 2, ou 3, destiné à une utilisation dans la détection de la présence d'un ou de plusieurs antigènes choisis dans le groupe constitué d'un antigène du VIH-1 et d'un antigène du VIH-2, dans un échantillon à tester, ledit procédé comprenant :
la mise en contact dudit échantillon à tester avec ledit anticorps qui se lie à la protéine p24 du virus de l'immunodéficience humaine 1 et à la protéine p26 du virus de l'immunodéficience humaine 2 pendant un temps et dans des conditions suffisantes pour la formation d'un complexe anticorps/antigène ; et
la détection dudit complexe anticorps/antigène, où la présence dudit complexe indique la présence d'au moins l'antigène du VIH-1 ou du VIH-2 dans ledit échantillon à tester.

6. Anticorps destiné à l'utilisation selon la revendication 5, où ledit anticorps est marqué et ladite détection comprend la détection dudit marqueur.

7. Anticorps destiné à l'utilisation selon la revendication 5, comprenant en outre :
la mise en contact d'un conjugué avec ledit complexe anticorps/antigène pendant un temps et dans des conditions suffisantes pour permettre audit conjugué de se lier à l'antigène lié composant ledit complexe, où ledit conjugué comprend un second anticorps fixé à un composé générateur de signal capable de générer un signal détectable ; et
la détection de la présence de l'antigène présent dans ledit échantillon à tester par la détection d'un signal généré par ledit composé générateur de signal, la présence ou la force dudit signal indiquant la présence d'au moins un antigène choisi dans le groupe constitué de l'antigène du VIH-1 et de l'antigène du VIH-2 dans ledit échantillon à tester.

8. Kit pour la détection de la présence d'un ou de plusieurs antigènes choisis dans le groupe constitué de l'antigène du VIH-1 et de l'antigène du VIH-2, dans un échantillon à tester, ledit kit comprenant :
un premier anticorps qui se lie à l'antigène p24 du VIH-1 et à l'antigène p26 du VIH-2 ; et/ou
un réactif indicateur comprenant un second anticorps qui se lie à l'antigène p24 du VIH-1 et à l'antigène p26 du VIH-2 auquel un composé générateur de signal est fixé ; dans lequel ledit premier anticorps et/ou ledit second anticorps est un anticorps selon l'une quelconque des revendications 1, 2, ou 3.

9. Anticorps isolé selon la revendication 1, qui comprend une chaîne lourde comprenant la séquence d'acides aminés de SEQ ID NO : 54 ou SEQ ID NO : 58 et une chaîne légère comprenant la séquence d'acides aminés de SEQ ID NO : 55 ou SEQ ID NO : 59.

10. Anticorps isolé selon la revendication 1, qui comprend une chaîne lourde comprenant la séquence d'acides aminés de SEQ ID NO : 46 ou SEQ ID NO : 50 et une chaîne légère comprenant la séquence d'acides aminés de SEQ ID NO : 47 ou SEQ ID NO : 51.
